(19) Europäisches Patentamt
European Patent Office
Office européen des brevets

(11) EP 2 089 386 B1

(12) **EUROPEAN PATENT SPECIFICATION**

(45) Date of publication and mention of the grant of the patent:
**08.08.2012 Bulletin 2012/32**

(21) Application number: **07859227.6**

(22) Date of filing: **07.11.2007**

(51) Int Cl.:
***C07D 413/14** (2006.01)* ***A61K 31/422** (2006.01)*
***C07D 413/04** (2006.01)*

(86) International application number:
**PCT/IB2007/004160**

(87) International publication number:
**WO 2008/056259 (15.05.2008 Gazette 2008/20)**

(54) **OXAZOLE DERIVATIVES AS POSITIVE ALLOSTERIC MODULATORS OF METABOTROPIC GLUTAMATE RECEPTORS**

OXAZOLDERIVATE ALS POSITIVE ALLOSTERISCHE MODULATOREN METABOTROPER GLUTAMATREZEPTOREN

DERIVES D'OXAZOLE UTILISES EN TANT QUE MODULATEURS ALLOSTERIQUES POSITIFS DES RECEPTEURS METABOTROPIQUES DU GLUTAMATE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **07.11.2006 GB 0622202**

(43) Date of publication of application:
**19.08.2009 Bulletin 2009/34**

(73) Proprietor: **ADDEX Pharma S.A.**
**1228 Plan-les-Ouates (CH)**

(72) Inventors:
- **LE POUL, Emmanuel**
  **1228 Plan-les-Ouates (CH)**
- **PALOMBI, Giovanni**
  **20021 Milan (IT)**
- **ROCHER, Jean-Philippe**
  **1228 Plan les Ouates (CH)**

(74) Representative: **Davies, Jonathan Mark**
**Reddie & Grose**
**16 Theobalds Road**
**London WC1X 8PL (GB)**

(56) References cited:
**WO-A-00/18744** **WO-A-2005/044797**
**WO-A-2006/048771** **WO-A-2007/111323**

- **ABBADY ET AL.: "Synthesis & Antimicrobial Activity of 2-Arylideneamino-4-phenyloxazoles, Azetidinones, Thiazolidinones, Thiazolines & Some Spiroheterocycles" INDIAN JOURNAL OF CHEMISTRY, vol. 27B, no. 1, 1988, pages 90-92, XP009100435**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

FIELD OF THE INVENTION

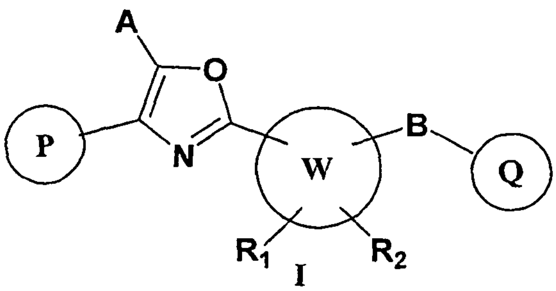

I

The present invention provides new compounds of formula I as positive allosteric modulators of metabotropic receptors - subtype 5 ("mGluR5") which are useful for the treatment or prevention of central nervous system disorders such as for example: cognitive decline, both positive and negative symptoms in schizophrenia as well as other disorders in which the mGluR5 subtype of glutamate metabotropic receptor is involved. The invention is also directed to pharmaceutical compounds and compositions in the prevention or treatment of such diseases in which mGluR5 is involved.

BACKGROUND OF THE INVENTION

Glutamate, the major amino-acid transmitter in the mammalian central nervous system (CNS), mediates excitatory synaptic neurotransmission through the activation of ionotropic glutamate receptors receptor-channels (iGluRs, namely NMDA, AMPA and kainate) and metabotropic glutamate receptors (mGluRs). iGluRs are responsible for fast excitatory transmission (Nakanishi S et al., (1998) Brain Res. Rev., 26:230-235) while mGluRs have a more modulatory role that contributes to the fine-tuning of synaptic efficacy. Glutamate performs numerous physiological functions such as long-term potentiation (LTP), a process believed to underlie learning and memory but also cardiovascular regulation, sensory perception, and the development of synaptic plasticity. In addition, glutamate plays an important role in the patho-physiology of different neurological and psychiatric diseases, especially when an imbalance in glutamatergic neurotransmission occurs.

The mGluRs are seven-transmembrane G protein-coupled receptors. The eight members of the family are classified into three groups.(Groups I, II & III) according to their sequence homology and pharmacological properties (Schoepp DD et al. (1999) Neuropharmacology, 38:1431-1476). Activation of mGluRs lead to a large variety of intracellular responses and activation of different transductional cascades. Among mGluR members, the mGluR5 subtype is of high interest for counterbalancing the deficit or excesses of neurotransmission in neuropsychatric diseases. mGluR5 belongs to Group I and its activation initiates cellular responses through G-protein mediated mechanisms. mGluR5 is coupled to phospholipase C and stimulates phosphoinositide hydrolysis and intracellular calcium mobilization.

mGluR5 proteins have been demonstrated to be localized in post-synaptic elements adjacent to the post-synaptic density (Lujan R et al. (1996) Eur. J. Neurosci., 8:1488-500; Lujan R et al. (1997) J. Chem. Neuroanat., 13: 219-41) and are rarely detected in the pre-synaptic elements (Romano C et al. (1995) J. Comp. Neurol., 355:455-69). mGluR5 receptors can therefore modify the post-synaptic responses to neurotransmitter or regulate neurotransmitter release.

In the CNS, mGluR5 receptors are abundant mainly throughout the cortex, hippocampus, caudate-putamen and nucleus accumbens. As these brain areas have been shown to be involved in emotion, motivational processes and in numerous aspects of cognitive function, mGluR5 modulators are predicted to be of therapeutic interest.

A variety of potential clinical indications have been suggested to be targets for the development of subtype selective mGluR modulators. These include epilepsy, neuropathic and inflammatory pain, numerous psychiatric disorders (eg anxiety, depression, schizophrenia and related psychotic disorders), movement disorders (eg Parkinson disease), neuroprotection (stroke and head injury), migraine and addiction/drug dependency (for reviews, see Bordi F and Ugolini A. (1999) Prog. Neurobiol., 59:55-79; Brauner-Osborne H et al. (2000) J. Med. Chem., 43:2609-45;; Spooren W et al. (2003) Behav. Pharmacol., 14:257-77; Marino MJ and Conn PJ. (2006) Curr. Opin. Pharmacol., 6: 98-102)

The hypothesis of hypofunction of the glutamatergic system as reflected by NMDA receptor hypofunction as a putative cause of schizophrenia has received increasing support over the past few years (Carlsson A et al. (2001) Annu. Rev. Pharmacol. Toxicol., 41:237-260 for a review; Goff DC and Coyle JT (2001) Am. J. Psychiatry, 158:

1367-1377). Evidence implicating dysfunction of glutamatergic neurotransmission is supported by the finding that antagonists of the NMDA subtypes of glutamate receptor can reproduce the full range of symptoms as well as the physiologic manifestation of schizophrenia such as hypofrontality, impaired prepulse inhibition and enhanced subcortical dopamine release. In addition, clinical studies have suggested that mGluR5 allele frequency is associated with schizophrenia among certain cohorts (Devon RS et al. (2001) Mol. Psychiatry., 6:311-4) and that an increase in mGluR5 message has been found in cortical pyramidal cells layers of schizophrenic brain (Ohnuma T et al. (1998) Brain Res. Mol. Brain Res., 56:207-17).

The involvement of mGluR5 in neurological and psychiatric disorders is supported by evidence showing that in vivo activation of group I mGluRs induces a potentiation of NMDA receptor function in a variety of brain regions mainly through the activation of mGluR5 receptors (Awad H. et al. (2000) J. Neurosci., 20:7871-7879; Mannaioni G. et al. (2001) Neuroscience., 21:5925-34; Pisani A et al. (2001) Neuroscience, 106:579-87; Benquet P. et al (2002) J. Neurosci., 22: 9679-86).

The role of glutamate in memory processes also has been firmly established during the past decade (Martin S.J. et al. (2000) Annu. Rev. Neurosci., 23:649-711; Baudry M. and Lynch G. (2001) Neurobiol. Learn. Mem., 76:284-297). The use of mGluR5 null mutant mice have strongly supported a role of mGluR5 in learning and memory. These mice show a selective loss in two tasks of spatial learning and memory, and reduced CA1 LTP (Lu et al. (1997) J. Neurosci., 17:5196-5205; Jia Z. et al. (2001) Physiol. Behav., 73:793-802; Schulz B et al. (2001) Neuropharmacology, 41:1-7; Rodrigues et al. (2002) J. Neurosci., 22:5219-5229).

The finding that mGluR5 is responsible for the potentiation of NMDA receptor mediated currents raises the possibility that agonists of this receptor could be useful as cognitive-enhancing agents, but also as novel antipsychotic agents that act by selectively enhancing NMDA receptor function.

The activation of NMDARs could potentiate hypofunctional NMDARs in neuronal circuitry relevant to schizophrenia. Recent in vivo data strongly suggest that mGluR5 activation may be a novel and efficacious approach to treat cognitive decline and both positive and negative symptoms in schizophrenia (Kinney GG et al. (2003) J. Pharmacol. Exp. Ther., 306(1):116-123; Lindsley et al. (2006) Curr. Top. Med. Chem 6:771-785).

mGluR5 receptor is therefore being considered as a potential drug target for treatment of psychiatric and neurological disorders including treatable diseases in this connection are anxiety disorders, attentional disorders, eating disorders, mood disorders, psychotic disorders, cognitive disorders, personality disorders and substance-related disorders.

Most of the current modulators of mGluR5 function have been developed as structural analogues of glutamate, quisqualate or phenylglycine (Schoepp DD et al. (1999) Neuropharmacology, 38:1431-1476) and it has been very challenging to develop in vivo active and selective mGluR5 modulators acting at the glutamate binding site. A new avenue for developing selective modulators is to identify molecules that act through allosteric mechanisms, modulating the receptor by binding to site different from the highly conserved orthosteric binding site.

Positive allosteric modulators of mGluRs have emerged recently as novel pharmacological entities offering this attractive alternative. This type of molecule has been discovered for mGluR1, mGluR2, mGluR4, mGluR5, mGluR7 and mGluR8 (Knoflach F. et al. (2001) Proc. Natl. Acad. Sci. USA., 98:13402-13407; Johnson K et al. (2002) Neuropharmacology, 43:291; O'Brien J.A. et al. (2003) Mol. Pharmacol., 64:731-40 ; Johnson M.P. et al. (2003) J. Med. Chem., 46:3189-92; Marino M.J. et al. (2003) Proc. Natl. Acad. Sci. USA., 100:13668-73; Mitsukawa K. et al. (2005) Proc Natl Acad Sci U S A 102(51):18712-7; Wilson J. et al. (2005) Neuropharmacology 49:278; for a review see Mutel V. (2002) Expert Opin. Ther. Patents, 12:1-8; Kew J.N. (2004) Pharmacol. Ther., 104(3):233-44; Johnson M.P. et al. (2004) Biochem. Soc. Trans., 32:881-7; recently Ritzen A., Mathiesen, J.M., and Thomsen C. (2005) Basic Clin. Pharmacol. Toxicol. 97:202-13). DFB and related molecules were described as in vitro mGluR5 positive allosteric modulators but with low potency (O'Brien JA et al. (2003) Mol. Pharmacol., 64:731-40). Benzamide derivatives have been patented (WO 2004/087048; O'Brien JA (2004) J. Pharmacol. Exp. Ther., 309:568-77) and recently aminopyrazole derivatives have been disclosed as mGluR5 positive allosteric modulators (Lindsley et al. (2004) J. Med. Chem., 47:5825-8; WO 2005/087048). Among aminopyrazole derivatives, CDPPB has shown in vivo activity antipsychotic-like effects in rat behavioral models (Kinney GG et al. (2005) J. Pharmacol. Exp. Ther., 313:199-206). Recently, intracerebroventricular application of DFB has been shown to result in a marked improvement in spatial alternation retention when it was tested 24 h after training, suggesting that the enhancement of intrinsic mGluR5 activity immediately during a critical period for memory consolidation have a positive impact on long-term memory retention in rats (Balschun D., Zuschratter W. and Wetzel W. (2006) Neuroscience 142:691-702). These reports are consistent with the hypothesis that allosteric potentiation of mGluR5 may provide a novel approach for development of antipsychotic or cognitive enhancers agents. Recently two novel series of positive allosteric modulators of mGluR5 receptors have been disclosed (WO05044797A1, WO06048771A1). Further references are :KO 2007/111323 WO 00/18744 and ABBADY et al., "Indian Journal of Chemistry", vol.27B n°1, 1988, pages 90-92.

The compounds of the invention demonstrate advantageous properties over compounds of the prior art. Improvements have been observed in one or more of the following characteristics of the compounds of the invention: the

potency on the target, the selectivity for the target, the solubility, the bioavailability, the brain penetration, and the activity in behavioural models of psychiatric and neurological disorders.

DETAILED DESCRIPTION OF THE INVENTION

According to the present invention, there are provided new compounds of the general formula I-A

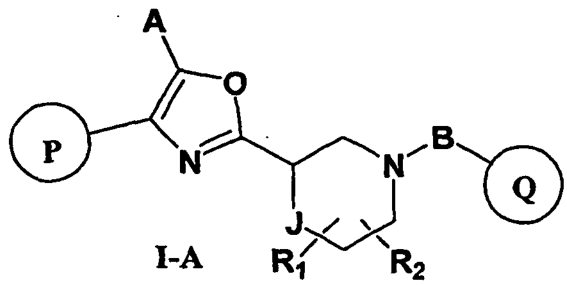

I-A

Or pharmaceutically acceptable salts, hydrates or solvates of such compounds
Wherein

$R_1$ and $R_2$ represent independently hydrogen, -($C_1$-$C_6$)alkyl, -($C_2$-$C_6$)alkenyl, - ($C_2$-$C_6$)alkynyl, arylalkyl, heteroarylalkyl, hydroxy, amino, aminoalkyl, hydroxyalkyl, -($C_1$-$C_6$)alkoxy or $R_1$ and $R_2$ together can form a ($C_3$-$C_7$) cycloalkyl ring, a carbonyl bond C=O or a carbon double bond;

P and Q are each independently selected and denote a cycloalkyl, a heterocycloalkyl, an aryl or heteroaryl group of formula

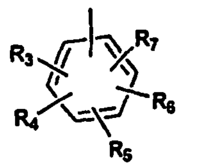 , 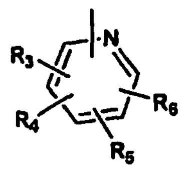 , 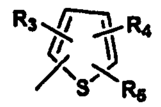 , 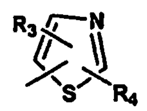 , 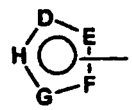

$R_3$, $R_4$, $R_5$, $R_6$, and $R_7$ independently are hydrogen, halogen, -$NO_2$, - ($C_1$-$C_6$)alkyl, -($C_3$-$C_6$)cycloalkyl, -($C_3$-$C_7$)cycloalkylalkyl, -($C_2$-$C_6$)alkenyl, -($C_2$-$C_6$)alkynyl, halo-($C_1$-$C_6$)alkyl, heteroaryl, heteroarylalkyl, arylalkyl, aryl, -$OR_8$, -$NR_8R_9$, -C(=$NR_{10}$)$NR_8R_9$, - $NR_8COR_9$, $NR_8CO_2R_9$, $NR_8SO_2R_9$, $NR_{10}CO$ $NR_8R_9$, -$SR_8$, -S(=O)$R_8$, -S(=O)$_2R_8$, -S(=O)$_2NR_8R_9$, -C(=O)$R_8$, -C(=O)-O-$R_8$, -C(=O)$NR_8R_9$, - C(=$NR_8$)$R_9$, or C(=$NOR_8$) $R_9$ substituents; wherein optionally two substituents are combined to the intervening atoms to form a bicyclic heterocycloalkyl, aryl or heteroaryl ring; wherein each ring is optionally further substituted with 1-5 independent halogen, -CN, -($C_1$-$C_6$)alkyl, -O-($C_0$-$C_6$)alkyl, -O-($C_3$-$C_7$)cycloalkylalkyl, -O(aryl), - O(heteroaryl), -O-(-$C_1$-$C_3$)alkylaryl, -O-($C_1$-$C_3$)alkylheteroaryl, -N((-$C_0$-$C_6$)alkyl)(($C_0$-$C_3$)alkylaryl) or N(($C_0$-$C_6$) alkyl)(($C_0$-$C_3$- )alkylheteroaryl) groups;
$R_8$, $R_9$, $R_{10}$ each independently is hydrogen, ($C_1$-$C_6$)alkyl, ($C_3$-$C_6$)cycloalkyl, ($C_3$-$C_7$)cycloalkylalkyl, ($C_2$-$C_6$)alkenyl, ($C_2$-$C_6$)alkynyl, halo-($C_1$-$C_6$)alkyl, heterocycloalkyl, heteroaryl, heteroarylalkyl, arylalkyl or aryl; any of which is optionally substituted with 1-5 independent halogen, -CN, -($C_1$-$C_6$)alkyl, -O-($C_0$-$C_6$) alkyl, -O-($C_3$-$C_7$)cycloalkylalkyl, -O(aryl), -O(heteroaryl), -N($C_0$-$C_6$-alkyl)$_2$,-N(($C_0$-$C_6$)alkyl)(($C_3$-$C_7$-)cycloalkyl) or -N(($C_0$-$C_6$)alkyl)(aryl) substituents;
D, E, F, G and H represent independently -C($R_3$)=-, -C($R_3$)=C($R_4$)-,-C(=O)-,-C(=S)-, -O-, -N=, -N($R_3$)- or -S-;

A is hydrogen, ($C_1$-$C_6$)alkyl, ($C_3$-$C_6$)cycloalkyl, ($C_3$-$C_7$)cycloalkylalkyl, ($C_2$-$C_6$)alkenyl, ($C_2$-$C_6$)alkynyl, halo-($C_1$-$C_6$)alkyl, heterocycloalkyl, heteroaryl, heteroarylalkyl, arylalkyl or aryl; any of which is optionally substituted with 1-5 independent halogen, -CN, -($C_1$-$C_6$)alkyl, -O-($C_0$-$C_6$)alkyl, -O-($C_3$-$C_7$)cycloalkylalkyl, -O(aryl), -O(heteroaryl), -N(Co-$C_6$-alkyl)$_2$,-N(($C_0$-$C_6$)alkyl)(($C_3$-$C_7$-)cycloalkyl) or -N(($C_0$-$C_6$)alkyl)(aryl) substituents;

B represents a single bond, -C(=O)-($C_0$-$C_2$)alkyl-, -C(=O)-($C_2$-$C_6$)alkenyl-, -C(=O)-($C_2$-$C_6$)alkynyl-, -C

(=O)-O-, $-C(=O)NR_8-(C_0-C_2)alkyl-$, $-C(=NR_8)NR_9-S(=O)-(C_0-C_2)alkyl-$, $-S(=O)_2-(C_0-C_2)alkyl-$, -S (=O)$_2$NR$_8$-(C$_0$-C$_2$)alkyl-, $C(=NR_8)-(C_0-C_2)alkyl-$, $-C(=NOR_8)-(C_0-C_2)alkyl-$ or $-C(=NOR_8)NR_9-(C_0-C_2)$ alkyl-;

$R_8$ and $R_9$, independently are as defined above;

J represents a single bond, $-C(R_{11})(R_{12})$, -O-, $-N(R_{11})-$ or -S-;

$R_{11}$, $R_{12}$ independently are hydrogen, $-(C_1-C_6)alkyl$, $-(C_3-C_6)cycloalkyl$, $-(C_3-C_7)cycloalkylalky)$, $-(C_2-C_6)$ alkeny), $-(C_2-C_6)alkynyl$, halo($C_1$-$C_6$)alkyl, heteroaryl, heteroarylalkyl, arylalkyl or aryl; any of which is optionally substituted with 1-5 independent halogen, -CN, $-(C_1-C_6)alkyl$, $-O(C_0-C_6)alkyl$, $-O(C_3-C_7)$cycloalkylalkyl, -O(aryl), - O(heteroaryl), $-N((C_0-C_6)alkyl)((C_0-C_6)alkyl)$,$-N((C_0-C_6)alkyl)((C_3-C_7)cycloalkyl)$ or $-N((C_0-C_6)alkyl)(aryl)$ substituents;

Any N may be an N-oxide.

The present invention includes both possible stereoisomers and includes not only racemic compounds but the individual enantiomers as well.

For the avoidance of doubt it is to be understood that in this specification "($C_1$-$C_6$)" means a carbon group having 1, 2, 3, 4, 5 or 6 carbon atoms. "($C_0$-$C_6$)" means a carbon group having 0, 1, 2, 3, 4, 5 or 6 carbon atoms. In this specification "C" means a carbon atom.

In the above definition, the term "($C_1$-$C_6$)alkyl" includes group such as methyl, ethyl, propyl, isopropyl, butyl, isobutyl, sec-butyl, tert-butyl, pentyl, isopentyl, neopentyl, tert-pentyl, hexyl or the like.

"($C_2$-$C_6$)alkenyl" includes group such as ethenyl, 1-propenyl, allyl, isopropenyl, 1-butenyl, 3-butenyl, 4-pentenyl and the like.

"($C_2$-$C_6$)alkynyl" includes group such as ethynyl, propynyl, butynyl, pentynyl and the like.

"Halogen" includes atoms such as fluorine, chlorine, bromine and iodine.

"Cycloalkyl" refers to an optionally substituted carbocycle containing no heteroatoms, includes mono-, bi-, and tricyclic saturated carbocycles, as well as fused ring systems. Such fused ring systems can include on ring that is partially or fully unsaturated such as a benzene ring to form fused ring systems such as benzo fused carbocycles. Cycloalkyl includes such fused ring systems as spirofused ring systems. Examples of cycloalkyl include cyclopropyl, cyclobutyl, cyclopentyl, cyclohexyl, decahydronaphthalene, adamantane, indanyl, fluorenyl, 1,2,3,4-tetrahydronaphthalene and the like.

"Heterocycloalkyl" refers to an optionally substituted carbocycle containing at least one heteroatom selected independently from O, N, S. It includes mono-, bi-, and tricyclic saturated carbocycles, as well as fused ring systems. Such fused ring systems can include one ring that is partially or fully unsaturated such as a benzene ring to form fused ring systems such as benzo fused carbocycles. Examples of heterocycloalkyl include piperidine, piperazine, morpholine, tetrahydrothiophene, indoline, isoquinoline and the like.

"Aryl" includes ($C_6$-$C_{10}$)aryl group such as phenyl, 1-naphtyl, 2-naphtyl and the like.

"Arylalkyl" includes ($C_6$-$C_{10}$)aryl-($C_1$-$C_3$)alkyl group such as benzyl group, 1-phenylethyl group, 2-phenylethyl group, 1-phenylpropyl group, 2-phenylpropyl group, 3-phenylpropyl group, 1-naphtylmethyl group, 2-naphtylmethyl group or the like.

"Heteroaryl" includes 5-10 membered heterocyclic group containing 1 to 4 heteroatoms selected from oxygen, nitrogen or sulphur to form a ring such as furyl (furan ring), benzofuranyl (benzofuran ring), thienyl (thiophene ring), benzothiophenyl (benzothiophene ring), pyrrolyl (pyrrole ring), imidazolyl (imidazole ring), pyrazolyl (pyrazole ring), thiazolyl (thiazole ring), isothiazolyl (isothiazole ring), triazolyl (triazole ring), tetrazolyl (tetrazole ring), pyridil (pyridine ring), pyrazynyl (pyrazine ring), pyrimidinyl (pyrimidine ring), pyridazinyl (pyridazine ring), indolyl (indole ring), isoindolyl (isoindole ring), benzoimidazolyl (benzimidazole ring), purinyl group (purine ring), quinolyl (quinoline ring), phtalazinyl (phtalazine ring), naphtyridinyl (naphtyridine ring), quinoxalinyl (quinoxaline ring), cinnolyl (cinnoline ring), pteridinyl (pteridine ring), oxazolyl (oxazole ring), isoxazolyl (isoxazole ring), benzoxazolyl (benzoxazole ring), benzothiazolyly (benzothiaziole ring), furazanyl (furazan ring) and the like.

"Heteroarylalkyl" includes heteroaryl-($C_1$-$C_3$-alkyl) group, wherein examples of heteroaryl are the same as those illustrated in the above definition, such as 2-furylmethyl group, 3-furylmethyl group, 2-thienylmethyl group, 3-thienylmethyl group, 1-imidazolylmethyl group, 2-imidazolylmethyl group, 2-thiazolylmethyl group, 2-pyridylmethyl group, 3-pyridylmethyl group, 1-quinolylmethyl group or the like.

"Solvate" refers to a complex of variable stoechiometry formed by a solute (e.g. a compound of formula I) and a solvent. The solvent is a pharmaceutically acceptable solvent as water preferably; such solvent may not interfere with the biological activity of the solute.

"Optionally" means that the subsequently described event(s) may or may not occur, and includes both event (s), which occur, and events that do not occur.

The term "substituted" refers to substitution with the named substituent or substituents, multiple degrees of

substitution being allowed unless otherwise stated.

More preferred compounds of the present invention are compounds of formula I-B

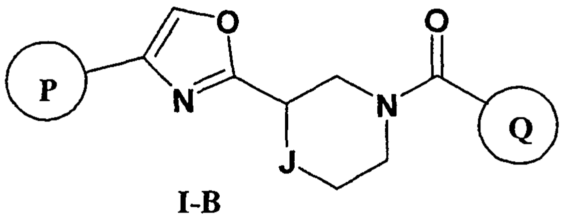

I-B

Or pharmaceutically acceptable salts, hydrates or solvates of such compounds
Wherein

P and Q are each independently selected and denote a cycloalkyl, a heterocycloalkyl, an aryl or heteroaryl group of formula

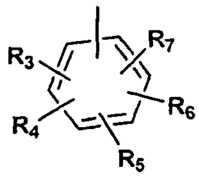 , 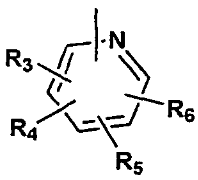 , 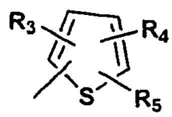 , 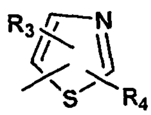 , 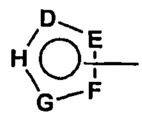

$R_3$, $R_4$, $R_5$, $R_6$, and $R_7$ independently are hydrogen, halogen, $-NO_2$, - $(C_1-C_6)$alkyl, $-(C_3-C_6)$cycloalkyl, $-(C_3-C_7)$cycloalkylalkyl, $-(C_2-C_6)$alkenyl, $-(C_2-C_6)$alkynyl, halo-$(C_1-C_6)$alkyl, heteroaryl, heteroarylalkyl, arylalkyl, aryl, $-OR_8$, $-NR_8R_9$, $-C(=NR_{10})NR_8R_9$, - $NR_8COR_9$, $NR_8CO_2R_9$, $NR_8SO_2R_9$, $-NR_{10}CO\ NR_8R_9$, $-SR_8$, $-S(=O)R_8$, $-S(=O)_2R_8$, $-S(=O)_2NR_8R_9$, $-C(=O)R_8$, $-C(=O)-O-R_8$, $-C(=O)NR_8R_9$, - $C(=NR_8)R_9$, or $C(=NOR_8)$ $R_9$ substituents; wherein optionally two substituents are combined to the intervening atoms to form a bicyclic heterocycloalkyl, aryl or heteroaryl ring; wherein each ring is optionally further substituted with 1-5 independent halogen, -CN, $-(C_1-C_6)$alkyl, $-O-(C_0-C_6)$alkyl, $-O-(C_3-C_7)$cycloalkylalkyl, -O(aryl),-O(heteroaryl), -O-$(-C_1-C_3)$alkylaryl, $-O-(C_1-C_3)$alkylheteroaryl, $-N((-C_0-C_6)$alkyl$)((C_0-C_3)$alkylaryl) or $-N((C_0-C_6)$alkyl$)((C_0-C_3-)$alkylheteroaryl) groups;

$R_8$, $R_9$, $R_{10}$ each independently is hydrogen, $-(C_1-C_6)$alkyl, $-(C_3-C_6)$cycloalkyl, $-(C_3-C_7)$cycloalkylalkyl, $-(C_2-C_6)$alkenyl, $-(C_2-C_6)$alkynyl, halo-$(C_1-C_6)$alkyl, heterocycloalkyl, heteroaryl, heteroarylalkyl, arylalkyl or aryl; any of which is optionally substituted with 1-5 independent halogen, -CN, $-(C_1-C_6)$alkyl,$-O-(C_0-C_6)$alkyl, $-O-(C_3-C_7)$cycloalkylalkyl, -O(aryl), -O(heteroaryl), $-N(C_0-C_6$-alkyl$)_2$,$-N((C_0-C_6)$alkyl$)((C_3-C_7-)$cycloalkyl) or $N((C_0-C_6)$alkyl)(aryl) substituents;

D, E, F, G and H represent independently $-C(R_3)=$, $-C(R_3)=C(R_4)-$,-C(=O)-,-C(=S)-, -O-, -N=, $-N(R_3)-$ or -S-;

J represents a single bond, $-C(R_{11})(R_{12})$, -O-, $-N(R_{11})-$ or -S-;

$R_{11}$, $R_{12}$ independently are hydrogen, $-(C_1-C_6)$alkyl, $-(C_3-C_6)$cycloalkyl, $-(C_3-C_7)$cycloalkylalkyl, $-(C_2-C_6)$ alkenyl, $-(C_2-C_6)$alkynyl, halo$(C_1-C_6)$alkyl, heteroaryl, heteroarylalkyl, arylalkyl or aryl; any of which is optionally substituted with 1-5 independent halogen, -CN, $-(C_1-C_6)$alkyl, $-0(C_0-C_6)$alkyl, $-O(C_3-C_7)$cycloalkylalkyl, -O(aryl), - O(heteroaryl), $-N((C_0-C_6)$alkyl$)((C_0-C_6)$alkyl),$-N((C_0-C_6)$alkyl$)((C_3-C_7)$cycloalkyl) or $-N((C_0-C_6)$alkyl)(aryl) substituents;

Any N may be an N-oxide.

The present invention includes both possible stereoisomers and includes not only racemic compounds but the individual enantiomers as well.

Specifically preferred compounds are:

(4-Fluoro-phenyl)-{(S)-3-[4-(4-fluoro-1H-pyrrol-2-yl)-oxazol-2-yl]-piperidin-1-yl}-methanone
(6-Fluoro-pyridin-3-yl)-{(S)-3-[4-(4-fluoro-1H-pyrrol-2-yl)-oxazol-2-yl]-piperidin-1-yl}-methanone
(4-Fluoro-phenyl)-{(S)-3-[4-(4-fluorophenyl)-oxazol-2-yl]-piperidin-1-yl}-methanone
(6-Fluoro-pyridin-3-yl)-{(S)-3-[4-(4-fluoro-phenyl)-oxazol-2-yl]-piperidin-1-yl}-methanone
(2-Fluoro-pyridin-4-yl)-{(S)-3-[4-(4-fluoro-phenyl)-oxazol-2-yl]-piperidin-1-yl}-methanone

(3-Fluoro-pyridin-4-yl)-{(S)-3-[4-(4-fluoro-phenyl)-oxazol-2-yl]-piperidin-1-yl}-methanone
(S)-(3-(4-(4-Fluoro-phenyl)-oxazol-2-yl)-piperidin-1-yl)(5-methyl-isoxazol-4-yl)-methanone
(S)-(4-Fluoro-phenyl)(3-(4-(pyridin-2-yl)-oxazol-2-yl)-piperidin-1-yl)-methanone
(S)-(3,4-Difluoro-phenyl)(3-(4-(pyridin-2-yl)-oxazol-2-yl)-piperidin-1-yl)-methanone
(S)-(4-Fluoro-phenyl)(3-(4-(5-fluoro-pyridin-2-yl)-oxazol-2-yl)-piperidin-1-yl)-methanone
(S)-(4-Fluoro-phenyl)(3-(4-(2-fluoro-phenyl)-oxazol-2-yl)-piperidin-1-yl)-methanone
(S)-(3-(4-(2-Fluoro-phenyl)-oxazol-2-yl)-piperidin-1-yl)(6-fluoro-pyridin-3-yl)-methanone
(S)-(3-(4-(2-Fluoro-phenyl)-oxazol-2-yl)-piperidin-1-yl)(2-fluoro-pyridin-4-yl)-methanone
(S)-(3-(4-(2,4-Difluoro-phenyl)-oxazol-2-yl)-piperidin-1-yl)(4-fluoro-phenyl)-methanone
(S)-(3-(4-(2,4-Difluoro-phenyl)-oxazol-2-yl)-piperidin-1-yl)(6-fluoro-pyridin-3-yl)-methanone
(S)-(3-(4-(2,4-Difluoro-phenyl)-oxazol-2-yl)-pipendin-l-yl)(2-fluoro-pyridin-4-yl)-methanone.

The present invention relates to the pharmaceutically acceptable acid addition salts of compounds of the formula I or pharmaceutically acceptable carriers or excipients.

The present invention relates to pharmaceutical compositions which provide from about 0.01 to 1000 mg of the active ingredient per unit dose. The compositions may be administered by any suitable route: for example orally in the form of capsules or tablets, parenterally in the form of solutions for injection, topically in the form of onguents or lotions, ocularly in the form of eye-lotion, rectally in the form of suppositories.

The pharmaceutical formulations of the invention may be prepared by conventional methods in the art; the nature of the pharmaceutical composition employed will depend on the desired route of administration. The total daily dose usually ranges from about 0.05 - 2000 mg.

METHODS OF SYNTHESIS

Compounds of general formula I may be prepared by methods known in the art of organic synthesis as set forth in part by the following synthesis schemes. In all of the schemes described below, it is well understood that protecting groups for sensitive or reactive groups are employed where necessary in accordance with general principles of chemistry. Protecting groups are manipulated according to standard methods of organic synthesis (Green T.W. and Wuts P.G.M. (1991) Protecting Groups in Organic Synthesis, John Wiley et Sons). These groups are removed at a convenient stage of the compound synthesis using methods that are readily apparent to those skilled in the art. The selection of process as well as the reaction conditions and order of their execution shall be consistent with the preparation of compounds of formula **I**.

The compound of formula I may be represented as a mixture of enantiomers, which may be resolved into the individual pure *R*- or *S*-enantiomers. If for instance, a particular enantiomer of the compound of formula I is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group such as amino, or an acidic functional group such as carboxyl, this resolution may be conveniently performed by fractional crystallization from various solvents, of the salts of the compounds of formula I with optical active acid or by other methods known in the literature, e.g. chiral column chromatography.

Resolution of the final product, an intermediate or a starting material may be performed by any suitable method known in the art as described by Eliel E.L., Wilen S.H. and Mander L.N. (1984) Stereochemistry of Organic Compounds, Wiley-Interscience.

Many of the heterocyclic compounds of formula I can be prepared using synthetic routes well known in the art (Katrizky A.R. and. Rees C.W. (1984) Comprehensive Heterocyclic Chemistry, Pergamon Press).

The product from the reaction can be isolated and purified employing standard techniques, such as extraction, chromatography, crystallization, distillation, and the like.

The compounds of formula **I-A** may be prepared according to the synthetic sequences illustrated in the Schemes 1 and 2.

Wherein

P and Q each independently is aryl or heteroaryl as described above
B represents -C(=O)-$C_0$-$C_2$-alkyl-; -S(=O)2-$C_0$-$C_2$-alkyl-.
J is CH2 and A, R1 and R2 are H,

Scheme 1

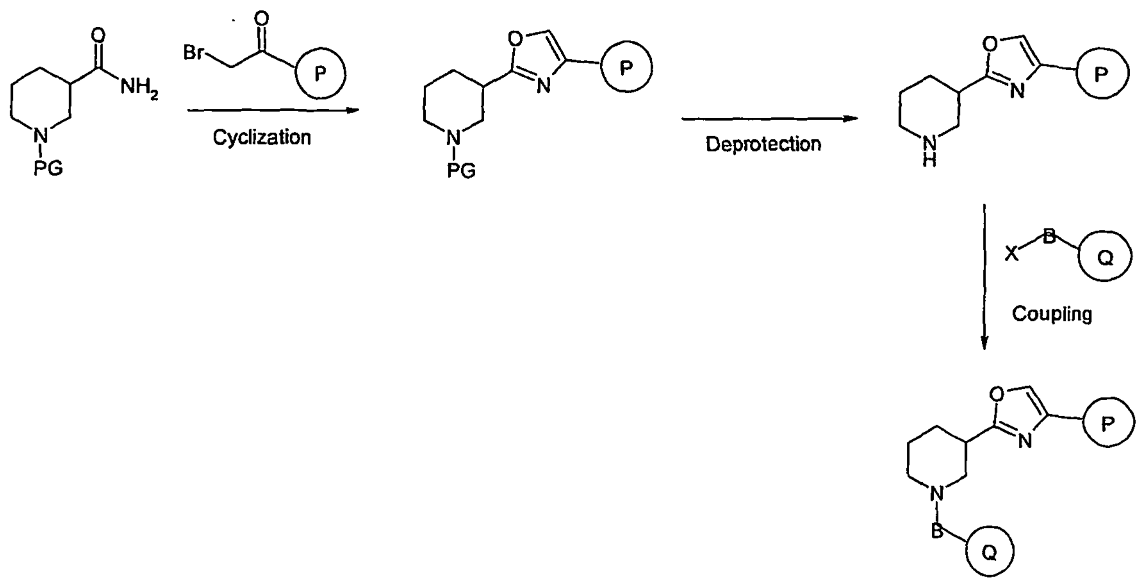


The precursor N-protected primary amide can be prepared using methods readily apparent to those skilled in the art, starting from N-protected-piperidine-3-carboxylic acid.

The precursor α-bromo-ketone derivatives described above are prepared according to synthetic routes well known in the art.

In the Scheme 1, PG is an amino protecting group such as Benzyloxycarbonyl, Ethoxycarbonyl, Benzyl and the like.

Thus, a primary amide (for example, (S)-3-Carbamoyl-piperidine-1-carboxylic acid benzyl ester) is reacted with an α-bromo-ketone derivative under neutral or basic conditions such as triethylamine, diisopropyl-ethylamine and the like, in a suitable solvent (*e.g.*N-methyl-pyrrolidone (NMP), dimethylformamide (DMF), xylene and the like) or without solvent, but simply mixing the primary amide and the α-bromo-ketone. The reaction typically proceeds by allowing the reaction temperature to warm slowly from ambient temperature to a temperature range of 100°C up to 150°C inclusive, for a time in the range of about 1 hour up to 48 hours inclusive. The reaction may be conducted under conventional heating (using an oil bath) or under microwaves heating. The reaction may be conducted in an open vessel or in a sealed tube.

As shown in the Scheme 1, protecting groups PG are removed using standard methods.

In the Scheme 1, B is as defined above, X is halogen or -OH. For example, in the case where X is halogen, the piperidine derivative is reacted with an aryl or heteroaryl acyl chloride using methods that are readily apparent to those skilled in the art. The reaction may be promoted by a base such as triethylamine, diisopropylamine, pyridine in a suitable solvent (*e.g.* tetrahydrofuran, dichloromethane). The reaction typically proceeds by allowing the reaction temperature to warm slowly from 0°C up to ambient temperature for a time in the range of about 4 up to 12 hours. In the case where X is -OH, the coupling reaction may be promoted by coupling agents known in the art of organic synthesis such as EDCI (1-(3-Dimethylaminopropyl)-3-ethylcarbodiimide), DCC (N,N'-Dicyclohexyl-carbodiimide) or by polymer-supported coupling agents such as polymer-supported carbodiimide (PS-DCC, ex Argonaut Technologies), in the presence of a suitable base such as triethylamine, diisopropyl-ethylamine, in a suitable solvent (e.g. tetrahydrofuran, dichloromethane, N,N-dimethylformamide, dioxane). Typically, a co-catalyst such as HOBT (1-Hydroxy-benzotriazole), HOAT (1-Hydroxy-7-azabenzotriazole) and the like may also be present in the reaction mixture. The reaction typically proceeds at ambient temperature for a time in the range of about 2 hours up to 24 hours.

Scheme 2

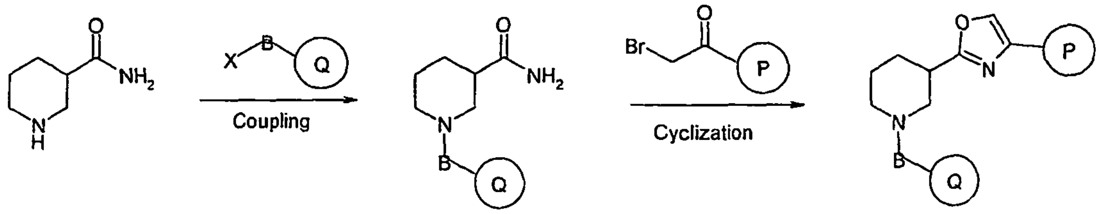


As an alternative synthetic route to obtain these derivatives, the pathway described in the Scheme 2 can be used. Thus, a primary amide like (S)-Piperidine-3-carboxylic acid amide (which can be easily prepared using methods that are readily apparent to those skilled in the art, starting from piperidine-3-carboxylic acid) can be reacted with an aryl or heteroaryl acyl chloride using methods that are readily apparent to those skilled in the art. The reaction may be promoted by a base such as triethylamine, diisopropylamine, pyridine in a suitable solvent (*e.g.* tetrahydrofuran, dichloromethane). The reaction typically proceeds by allowing the reaction temperature to warm slowly from 0°C up to ambient temperature for a time in the range of about 4 up to 12 hours. Alternatively, in the case where X is -OH, the coupling reaction may be promoted by coupling agents known in the art of organic synthesis such as EDCI (1-(3-Dimethylaminopropyl)-3-ethylcarbodiimide), DCC (N,N'-Dicyclohexyl-carbodiimide) or by polymer-supported coupling agents such as polymer-supported carbodiimide (PS-DCC, ex Argonaut Technologies), in the presence of a suitable base such as triethylamine, diisopropyl-ethylamine, in a suitable solvent (e.g. tetrahydrofuran, dichloromethane, N,N-dimethylformamide, dioxane). Typically, a co-catalyst such as HOBT (1-Hydroxy-benzotriazole), HOAT (1-Hydroxy-7-azabenzotriazole) and the like may also be present in the reaction mixture. The reaction typically proceeds at ambient temperature for a time in the range of about 2 hours up to 24 hours.

The cyclization step can be performed then as described above and in Scheme 1.

The compounds of Formula **I** which are basic in nature can form a wide variety of different pharmaceutically acceptable salts with various inorganic and organic acids. These salts are readily prepared by treating the base compounds with a substantially equivalent amount of the chosen mineral or organic acid in a suitable organic solvent such as methanol, ethanol or isopropanol (see Stahl P.H., Wermuth C.G., Handbook of Pharmaceuticals Salts, Properties, Selection and Use, Wiley, 2002).

The following non-limiting examples are intended to illustrate the invention. The physical data given for the compounds exemplified is consistent with the assigned structure of those compounds.

EXAMPLES

Unless otherwise noted, all starting materials were obtained from commercial suppliers and used without further purification.

Specifically, the following abbreviation may be used in the examples and throughout the specification.

| | |
|---|---|
| g (grams) | rt (room temperature) |
| mg (milligrams) | MeOH (methanol) |
| mL (millilitres) | |
| μl (microliters) | Hz (Hertz) |
| M (molar) | LCMS (Liquid Chromatography Mass Spectrum) |
| MHz (megahertz) | HPLC (High Pressure Liquid Chromatography) |
| mmol (millimoles) | NMR (Nuclear Magnetic Resonance) |
| min (minutes) | 1H (proton) |
| AcOEt (ethyl acetate) | $Na_2SO_4$ (sodium sulphate) |
| $K_2CO_3$ (potassium carbonate) | $MgSO_4$ (magnesium sulphate) |
| $CDCl_3$ (deuteriated chloroform) | HOBT (1-hydroxybenzotriazole) |

(continued)

| | |
|---|---|
| EDCI.HCl (1-3(Dimethylaminopropyl)-3-ethylcarbodiimide, hydrochloride) | RT (Retention Time) |
| EtOH (ethyl alcohol) | NaOH (sodium hydroxide) |
| % (percent) | h (hour) |
| DCM (dichloromethane) | HCl (hydrochloric acid) |
| DIEA (diisopropyl ethyl amine) | n-BuLi (n-butyllithium) |
| Mp (melting point) | THF (tetrahydrofuran) |

All references to brine refer to a saturated aqueous solution of NaCl. Unless otherwise indicated, all temperatures are expressed in °C (degrees Centigrade). All reactions are conducted under an inert atmosphere at room temperature unless otherwise noted.

$^1$H NMR spectra were recorded on a Brucker 500MHz or on a Brucker 300MHz. Chemical shifts are expressed in parts of million (ppm, δ units). Coupling constants are in units of hertz (Hz) Splitting patterns describe apparent multiplicities and are designated as s (singlet), d (doublet), t (triplet), q (quadruplet), q (quintuplet), m (multiplet).

LCMS were recorded under the following conditions:

Method A) Waters Alliance 2795 HT Micromass ZQ. Column Waters XTerra MS C18 (50x4.6 mm, 2.5μm). Flow rate 1 ml/min Mobile phase: A phase = water/$CH_3CN$ 95/5 + 0.05% TFA, B phase = water/$CH_3CN$ = 5/95 + 0.05% TFA. 0-1 min (A: 95%, B: 5%), 1-4 min (A: 0%, B: 100%), 4-6 min (A: 0%, B: 100%), 6-6.1 min (A: 95%, B: 5%). T= 35°C; UV detection: Waters Photodiode array 996, 200-400nm.

Method B) Waters Alliance 2795 HT Micromass ZQ. Column Waters Symmetry C18 (75x4.6 mm, 3.5μm). Flow rate 1.5 ml/min. Mobile phase: A phase = water/$CH_3CN$ 95/5 + 0.05% TFA, B phase = water/$CH_3CN$ = 5/95 + 0.05% TFA. 0-0.5 min (A: 95%, B: 5%), 0.5-7 min (A: 0%, B: 100%), 7-8 min (A: 0%, B: 100%), 8-8.1 min (A: 95%, B: 5%). T= 35°C; UV detection: Waters Photodiode array 996, 200-400nm.

Method C) Waters Alliance 2795 HT Micromass ZQ. Column Waters Atlantis C18 (75x4.6 mm, 3.0μm). Flow rate 1.5 ml/min. Mobile phase: A phase = water/$CH_3CN$ 95/5 + 0.05% TFA, B phase = water/$CH_3CN$ = 5/95 + 0.05% TFA. 0-0.5 min (A: 95%, B: 5%), 5.5 min (A: 0%, B: 100%), 5.5-8 min (A: 0%, B: 100%), 8.1 min (A: 95%, B: 5%). T= 35°C; UV detection: Waters Photodiode array 996, 200-400nm.

Method D): UPLC system Waters Acquity, Micromass ZQ2000 Single quadrupole (Waters). Column 2.1*50mm stainless steel packed with 1.7μm Acquity UPLC-BEH; flow rate 0.50 ml/min; mobile phase: A phase = water/ acetonitrile 95/5 + 0.05% TFA, B phase = water/acetonitrile 5/95 + 0.05% TFA. 0-0.1min (A: 95%, B: 5%), 1.6min (A: 0%, B: 100%), 1.6-1.9min (A: 0%, B: 100%), 2.4min (A: 95%, B: 5%); UV detection wavelenght 254 nm.

Method E): Pump 1525u (Waters), 2777 Sample Manager, Micromass ZQ2000 Single quadrupole (Waters); PDA detector: 2996 (Waters). Column: Acquity UPLC-BEH C 18 50x2.1 mmx1.7um; flow rate 0.25 ml/min splitting ratio MS :waste/ 1:4; mobile phase: A phase = water/acetonitrile 95/5 + 0.1 % TFA, B phase = water/acetonitrile 5/95 + 0.1% TFA. 0-1.0min (A: 98%, B: 2%), 1.0-5.0min (A: 0%, B: 100%), 5.0-9.0min (A: 0%, B: 100%), 9.1-12min (A: 98%, B: 2%); UV detection wavelenght 254 nm; Injection volume: 5μl.

Method F) Waters Alliance 2795 HT Micromass ZQ. Column Waters Symmetry C18 (75x4.6 mm, 3.5μm). Flow rate 1.5 ml/min. Mobile phase: A phase = water/$CH_3CN$ 95/5 + 0.05% TFA, B phase = water/$CH_3CN$ = 5/95 + 0.05% TFA. 0-0.5 min (A: 95%, B: 5%), 0.5-7 min (A: 0%, B: 100%), 7-8 min (A: 0%, B: 100%), 8-8.1 min (A: 95%, B: 5%). T= 35°C; UV detection: Waters Photodiode array 996, 200-400nm.

Method G) Waters Alliance 2795 HT Micromass ZQ. Column Waters Symmetry C18 (75x4.6 mm, 3.5μm). Flow rate 1.5 ml/min. Mobile phase: A phase = water/$CH_3CN$ 95/5 + 0.05% TFA, B phase = water/$CH_3CN$ = 5/95 + 0.05% TFA.

0-0.1 min (A: 95%, B: 5%), 6 min (A: 0%, B: 100%), 6-8 min (A: 0%, B: 100%), 8.1 min (A: 95%, B: 5%). T= 35°C; UV detection: Waters Photodiode array 996, 200-400nm.

Method H): HPLC system: Waters Acquity, MS detector: Waters ZQ2000. Column: Acquity UPLC-BEH C18 50x2.1mmx1.7um; flow rate 0.6 ml/min; mobile phase: A phase = water/acetonitrile 95/5 + 0.1 % TFA, B phase = water/acetonitrile 5/95 + 0.1 % TFA. 0-0.25min (A: 98%, B: 2%), 3.30min (A: 0%, B: 100%), 3.3-4.0min (A: 0%, B: 100%), 4.1min (A: 98%, B: 2%); UV detection wavelength 254 nm; Injection volume: 1μl.

Method I): HPLC system: Waters Acquity, MS detector: Waters ZQ2000. Column: Acquity UPLC-BEH C18 50x2.1mmx1.7um; flow rate 0.4 ml/min; mobile phase: A phase = water/acetonitrile 95/5 + 0.1% formic acid, B phase = water/acetonitrile 5/95 + 0.1% formic acid. 0-0.5min (A: 98%, B: 2%), 1.5min (A: 90%, B: 10%), 5.0min (A:70%,

B: 30%), 7.0min (A: 0%, B: 100%), 7.0-8.0min (A: 0%, B: 100%), 8.1min (A: 98%, B: 2%), 9.5min (A: 98%, B: 2%); UV detection wavelength 254 nm; Injection volume: 1μl.
All mass spectra were taken under electrospray ionisation (ESI) methods.

Most of the reactions were monitored by thin-layer chromatography on 0.25mm Macherey-Nagel silica gel plates (60F-2254), visualized with UV light. Flash column chromatography was performed on silica gel (220-440 mesh, Fluka).
Melting point determination was performed on a Buchi B-540 apparatus.

Example 1

(4-Fluoro-phenyl)-{(S)-3-[4-(4-fluoro-1H-pyrrol-2-yl)-oxazol-2-yl]-piperidin-1-yl} - methanone

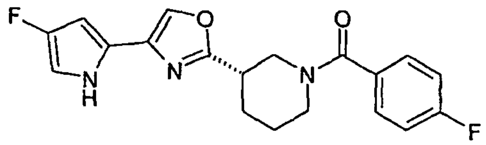

1 (A) (S)-3-Carbamoyl-piperidine-1-carboxylic acid tert-butyl ester

A solution of carbonyl-diimidazole (2.97 g, 18.3 mmol) in 50 mL of acetonitrile was added dropwise to a solution of (S)-N-Boc-nipecotic acid (4 g, 17.4 mmol) in acetonitrile (70 mL). After stirring at room temperature for 10 min, conc. $NH_4OH$ (aq.) (100 mL) was added and stirring was maintained for 1 h. The solvent was removed, the crude residue was dissolved in ethyl acetate and washed subsequently with citric acid (aq.), with water and then with brine. The organic layer was dried over sodium sulphate and evaporated under reduced pressure to afford (S)-3-Carbamoyl-piperidine-1-carboxylic acid tert-butyl ester, that was used for the next step without further purification.
Yield: quantitative; LCMS (RT): 3.31 min (Method F); MS (ES+) gave m/z: 229.0.

I (B) (S)-Piperidine-3-carboxylic acid amide hydrochloride

To a solution of (S)-3-carbamoyl-piperidine-1-carboxylic acid tert-butyl ester (2 g, 8.77 mmol), in dichloromethane (20 mL), 9 mL of 4N HCl (dioxane solution) were added at 0°C and the reaction mixture was allowed to warm at room temperature and stirred for 20 h. The solvent was evaporated under reduced pressure to give the title compound as a white solid, which was used for the next step without further purification.
Yield: quantitative; LCMS (RT): 0.76 min (Method C); MS (ES+) gave m/z: 128.9.

1 (C) (S)-1-(4-Fluoro-benzoyl)-piperidine-3-carboxylic acid amide

To a suspension of (S)-piperidine-3-carboxylic acid amide hydrochloride (8.77 mmol) in dry dichloromethane (10 mL), triethylamine (1.5 mL, 20 mmol) and 4-fluorobenzoyl chloride (1.1 mL, 9 mmol) were added dropwise at 0°C. The reaction mixture was allowed to warm at room temperature and stirred under nitrogen atmosphere for 24h. The solution was then treated with 0.2N NaOH (10 mL) and the phases were separated. The organic layer was washed with water (5 mL), with 0.2M HCl and with brine (5 mL), then was dried over $Na_2SO_4$ and evaporated under reduced pressure. The crude was purified by flash chromatography (silica gel, eluent gradient: from petroleum ether/ethyl acetate 100:0 to petroleum ether/ethyl acetate 0:100) to give 220 mg of the title compound.
Yield: 10%; LCMS (RT): 2.89 min (Method B); MS (ES+) gave m/z: 251.09.

1 (D) 4-Fluoro-1H-pyrrole-2-carboxylic acid methoxy-methyl-amide

A mixture of 4-fluoro-1H-pyrrole-2-carboxylic acid (500 mg, 3.8 mmol), O,N-Dimethyl-hydroxylamine hydrochloride (451 mg, 4.65 mmol), HOBT (891 mg, 5.812 mmol), EDC (1.110 g, 5.8 mmol) and TEA (2.174 ml, 15.5 mmol) in DCM (30 ml) was stirred at room temperature for 20 h. The solvent was evaporated under vacuum, the residue was partitioned between 5% $NaHCO_3$ (aq) and ethyl acetate. The organic phase was separated, dried over $Na_2SO_4$ and concentrated under vacuum. The crude was purified by flash chromatography (silica gel cartridge, eluent: ethyl acetate/petroleum ether 1:1) to give 555 mg of white solid.

Yield: 83%, LC-MS (RT): 1.02 min (Method D), MS (ES+) gave m/z: 173.0.

1(E) 4-Fluoro-1-(toluene-4-sulfonyl)-1H-pyrrole-2-carboxylic acid methoxy-methyl-amide

NaH (60% in mineral oil, 56 mg, 1.40 mmol) was added to a stirred solution of 4-fluoro-1H-pyrrole-2-carboxylic acid methoxy-methyl-amide (201 mg, 1.17 mmol) under nitrogen at room temperature. After 10 min, tosyl chloride (311 mg, 1.64 mmol) was added and the mixture was stirred for 1 h. $NH_4Cl_{sat}$ (aq) was added and the mixture was extracted with ethyl acetate. The organic phase was washed with brine, dried over $Na_2SO_4$ and concentrated. The crude was purified by flash chromatography (silica gel cartridge, eluent: ethyl acetate/petroleum ether 1:3) to give 300 mg of white solid.
Yield: 79%; LC-MS (RT): 1.43 min (Method D), MS (ES+) gave m/z: 326.9.

1(F) 1-[4-Fluoro-1-(toluene-4-sulfonyl)-1H-pyrrol-2-yl]-ethanone

A solution of methylmagnesiumbromide (3M sol THF, 0.443 ml, 1.33 mmol) was added to a stirred solution of 4-fluoro-1-(toluene-4-sulfonyl)-1H-pyrrole-2-carboxylic acid methoxy-methyl-amide (288 mg, 0.88 mmol) in dry THF (2 ml) at - 12 °C, under nitrogen. The mixture was stirred for 30 min at room temperature, then another portion of methylmagnesium bromide (3M sol THF, 0.443 ml, 1.33 mmol) was added. After 30 min, 0.5M HCl was added dropwise and the mixture was extracted twice with diethyl ether. The organic phase was dried over $Na_2SO_4$ and concentrated. The crude was purified by flash chromatography (silica gel cartridge, eluent: ethyl acetate/petroleum ether 1:5) to give 210 mg of white solid.
Yield: 85%; %; LC-MS (RT): 1.48 min (Method D), MS (ES+) gave m/z: 282.0.

1(G) 2-Bromo-1-[4-fluoro-1-(toluene-4-sulfonyl)-1H-pyrrol-2-yl]-ethanone

A mixture of 1-[4-fluoro-1-(toluene-4-sulfonyl)-1H-pyrrol-2-yl]-ethanone (50 mg, 0.178 mmol), pyridinium tribromide (63 mg, 0.196 mmol), HBr (48%, 0.076 ml) and glacial acetic acid (3.5 ml) was stirred at room temperature for 20 h. Volatiles were evaporated and the crude was purified by flash chromatography (silica gel cartridge, eluent: ethyl acetate/petroleum ether 1:9) to give 30 mg of viscous oil.
Yield: 47%; LCMS (RT): 5.9 min (Method D): MS (ES+) gave m/z: 359.9, 361.9.

1(H) (4-Fluoro-phenyl)-((S)-3-{4-[4-fluoro-1-(toluene-4-sulfonyl)-1H-pyrrol-2-yl]-oxazol-2-yl}-piperidin-1-yl)-methanone

2-Bromo-1-[4-fluoro-1-(toluene-4-sulfonyl)-1H-pyrrol-2-yl]-ethanone (120 mg, 0.333 mmol) and (S)-1-(4-fluoro-benzoyl)-piperidine-3-carboxylic acid amide (92 mg, 0.367 mmol), prepared as described in Example 1(C), were dissolved in dichloromethane (2 ml), the solvent was evaporated and the residue was heated at 125 °C for 6 h. After cooling to room temperature, 5 ml of acetonitrile were added and the mixture was treated with 2 eq of triethylamine and 0.5 eq of 4-fluoro-benzoylchloride. After 30 min, the solvent was evaporated and the crude was purified by flash chromatography (silica gel cartridge, eluent: ethyl acetate/petroleum ether 1:2) to give 43 mg of title compound.
Yield: 25%, LC-MS (RT): 1.73 min (Method D), MS (ES+) gave m/z: 511.8.

1(I) (4-Fluoro-phenyl)- {(S)-3-[4-(4-fluoro-1H-pyrrol-2-yl)-oxazol-2-yl]-piperidin-1-yl}-methanone.

A solution of TBAF (1M THF, 0.276 ml, 0.276 mmol) was added to a stirred solution of (4-fluoro-phenyl)-((S)-3-{4-[4-fluoro-1-(toluene-4-sulfonyl)-1H-pyrrol-2-yl]-oxazol-2-yl}-piperidin-1-yl)-methanone (47 mg, 0.092 mmol) in THF (4 ml). The mixture was heated at reflux for 5 min, the solvent was evaporated and the residue was partitioned between diethyl ether and water. The organic phase was separated and washed with 1N HCl and brine, dried over $Na_2SO_4$ and concentrated. The crude was purified by flash chromatography (silica gel cartridge, eluent: ethyl acetate/petroleum ether 1:1) to give 21 mg of title compound.
Yield: 64%; mp=136 °C; LCMS (RT): 2.22 min (Method E); MS (ES+) gave m/z: 358.1 (MH+).
$^1$H-NMR (DMSO-$d_6$, 353K), δ (ppm): 10.68 (s br, 1H); 8.04 (s, 1H); 7.46 (dd, 2H); 7.24 (dd, 2H); 6.62 (m, 1H); 6.17 (m, 1H); 4.21 (m, 1H); 3.80 (m, 1H); 3.36 (dd, 1H); 3.21 (ddd, 1H); 3.11 (ddd, 1H); 2.19 (m, 1H); 1.96-1.76 (m, 2H); 1.61 (m, 1H).

Example 2

(6-Fluoro-pyridin-3-yl)-{(S)-3-[4-(4-fluoro-1H-pyrrol-2-yl)-oxazol-2-yl]-piperidin-1-yl}-methanone

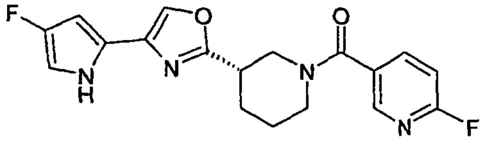

2(A) (S)-3-Carbamoyl-piperidine-1-carboxylic acid benzyl ester

Benzyl chloroformate (0.210 ml, 1.498 mmol) was added dropwise to a stirred solution of (S)-piperidine-3-carboxylic acid amide hydrochloride (234 mg, 1.427 mmol), prepared as described in Example 1(B), and triethylamine (0.5 ml, 3.567 mmol) in a mixture of dioxane (5 ml) and water (1 ml) at room temperature. After 30 min, the solvent was evaporated and the residue was dissolved in dichloromethane and washed with 1M $K_2CO_3$ (aq). The organic phase was dried over $Na_2SO_4$ and concentrated. The crude was purified by flash chromatography (silica gel cartridge, eluent: dichloromethane/methanol 20:1.5) to give 330 mg of white solid.
Yield: 88%; LCMS (RT): 3.4 min (Method A): MS (ES+) gave m/z: 263.1.

2(B) (S)-3-{4-[4-Fluoro-1-(toluene-4-sulfonyl)-1H-pyrrol-2-yl]-oxazol-2-yl}-piperidine-1-carboxylic acid benzyl ester

2-Bromo-1-[4-fluoro-1-(toluene-4-sulfonyl)-1H-pyrrol-2-yl]-ethanone (409 mg, 1.136 mmol), prepared as described in Example 1(G), and (S)-3-carbamoyl-piperidine-1-carboxylic acid benzyl ester (330 mg, 1.259 mmol), prepared as described in Example 2(A), were dissolved in dichloromethane (10 ml); the solvent was evaporated and the residue was heated at 125 °C for 6 h. The mixture was cooled to room temperature and dissolved in acetonitrile, then 0.244 ml of triethylamine and 0.073 ml of benzyl chloroformate were added. After stirring for 15 min, the solvent was evaporated, the residue was partitioned between dichloromethane and 1M $K_2CO_3$ (aq). The organic phase was separated, dried over $Na_2SO_4$ and concentrated. The crude was purified by flash chromatography (silica gel cartridge, eluent: ethyl acetate/petroleum ether 1:3) to give 230 mg of title compound.
Yield: 39%; LCMS (RT): 4.9 min (Method A): MS (ES+) gave m/z: 524.0.

2(C) (S)-3-[4-(4-Fluoro-1H-pyrrol-2-yl)-oxazol-2-yl]-piperidine-1-carboxylic acid benzyl ester

TBAF (1M sol. THF, 1.317 ml, 1.317 mmol) was added to a stirred solution of (S)-3-{4-[4-fluoro-1-(toluene-4-sulfonyl)-1H-pyrrol-2-yl]-oxazol-2-yl}-piperidine-1-carboxylic acid benzyl ester (230 mg, 0.439 mmol) in THF (15 ml). The mixture was heated at reflux for 2 min, the solvent was evaporated and the residue was partitioned between diethyl ether and 1N HCl. The organic phase was separated, washed with brine, dried over $Na_2SO_4$ and concentrated. The crude was purified by flash chromatography (silica gel cartridge, eluent: ethyl acetate/petroleum ether 2:3) to give 136 mg of title compound.
Yield: 84%; LC-MS (RT): 1.63 min (Method D), MS (ES+) gave m/z: 369.9.

2(D) (S)-3-[4-(4-Fluoro-1H-pyrrol-2-yl)-oxazol-2-yl]-piperidine

Pd/C (10%, 14 mg) was added to a stirred solution of (S)-3-[4-(4-fluoro-1H-pyrrol-2-yl)-oxazol-2-yl]-piperidine-1-carboxylic acid benzyl ester (136 mg, 0.368 mmol) and ammonium formate (114 mg, 1.84 mmol) in MeOH (14 ml). The mixture was heated at reflux for 5 min, cooled to room temperature and the catalyst was filtered off. The solution was concentrated, the residue was dissolved in DCM and washed with a solution of brine/1N $K_2CO_3$ 1/1. The organic phase was dried over $Na_2SO_4$ and concentrated to give 78 mg of beige solid.
Yield: 90%; LC-MS (RT): 0.91 min (Method D), MS (ES+) gave m/z: 236.0.

2(E) (6-Fluoro-pyridin-3-yl)-{(S)-3-[4-(4-fluoro-1H-pyrrol-2-yl)-oxazol-2-yl]-piperidin-1-yl}-methanone

A mixture of 6-fluoro-nicotinic acid (45 mg, 0.323 mmol), EDC (92 mg, 4.484 mmol), HOAT (66 mg, 0.484 mmol) and triethylamine (0.136 ml, 0.968 mmol) in dichloromethane (5 ml) was stirred for 30 min at room temperature; then a solution of (S)-3-[4-(4-fluoro-1H-pyrrol-2-yl)-oxazol-2-yl]-piperidine (76 mg, 0.323 mmol) in dichloromethane (5 ml) was added. After 22 h, the solvent was evaporated, the residue was partitioned between ethyl acetate and 5% $NaHCO_3$ (aq); the organic phase was separated, washed with brine, dried over $Na_2SO_4$ and concentrated. The crude was purified by flash chromatography (silica gel cartridge, eluent: ethyl acetate/petroleum ether 2: 1) to give 54 mg of pink solid.
Yield: 47%; mp=123 °C; [$\alpha_D$]= +104.0° (MeOH, c=1.000); LCMS (RT): 1.98 min (Method E); MS (ES+) gave m/z: 359.1 (MH+).

$^1$H-NMR (DMSO-$d_6$, 353K), δ (ppm): 10.68 (s br, 1H); 8.30 (m, 1H); 8.04 (s, 1H); 8.01 (ddd, 1H); 7.21 (ddd, 1H); 6.62 (m, 1H); 6.16 (m, 1H); 4.20 (m, 1H); 3.78 (m, 1H); 3.42 (dd, 1H); 3.28 (ddd, 1H); 3.15 (ddd, 1H); 2.19 (m, 1H); 2.00-1.77 (m, 2H); 1.65 (m, 1H).

Example 3

(4-Fluoro-phenyl)-{(S)-3-[4-(4-fluorophenyl)-oxazol-2-yl]-piperidin-1-yl}-methanone

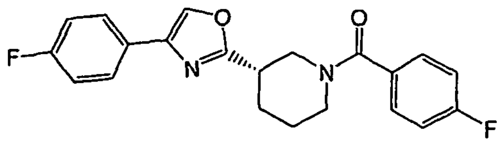

A solution of (S)-1-(4-fluoro-benzoyl)-piperidine-3-carboxylic acid amide (1.8 g, 7.19 mmol), prepared as described in Example 1(C), and 4-fluorophenacyl bromide (625 mg, 2.88 mmol) in dry N-methyl-2-pyrrolidinone (10 mL) was heated at 100°C for 14 h. The reaction mixture was cooled to room temperature, ethyl acetate was added and the organic layer was washed sequentially with water (twice) and with brine (twice). The organics were dried over sodium sulphate and evaporated under reduced pressure to afford a crude oil that was purified by flash chromatography (silica gel, eluent: petroleum ether/ethyl acetate 7:3). 350 mg of (4-fluoro-phenyl)-{(S)-3-[4-(4-fluorophenyl)-oxazol-2-yl]-piperidin-1-yl}-methanone were obtained as a yellow solid.
Yield: 33%; [$\alpha_D$]= +92.64° (c=0.9, $CH_3OH$); LCMS (RT): 3.26 min (Method H); MS (ES+) gave m/z: 369.1 (MH+).
$^1$H-NMR (DMSO-$d_6$, 353K), δ (ppm): 8.34 (s, 1H) 7.74-7.81 (m, 2H) 7.41-7.49 (m, 2H) 7.17-7.26 (m, 4H) 4.19 (dd, 1H) 3.77 (ddd, 1H) 3.45 (dd, 1H) 3.27 (ddd, 1H) 3.08-3.20 (m, 1H) 2.16-2.27 (m, 1H) 1.77-2.01 (m, 2H) 1.54-1.68 (m, 1H).

Example 4

(6-Fluoro-pyridin-3-yl)-{(S)-3-[4-(4-fluoro-phenyl)-oxazol-2-yl]-piperidin-1-yl}-methanone

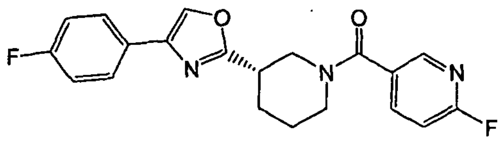

4(A) (S)-3-[4-(4-Fluoro-phenyl)-oxazol-2-yl]-piperidine-1-carboxylic acid benzyl ester

A solution of 4-fluorophenacyl bromide (217 mg, 1.0 mmol) and (S)-3-carbamoyl-piperidine-1-carboxylic acid benzyl ester (500 mg, 1.9 mmol), prepared as described in Example 2(A), in dry N-methyl-2-pyrrolidinone (5 mL) was heated at 150°C; for 3 h, under nitrogen atmosphere. The reaction mixture was cooled to room temperature, ethyl acetate was added and the organic layer was washed sequentially with water (twice), 0.2M NaOH (aq.), 0.2M HCl (aq.) and with brine (twice). The organics were dried over sodium sulphate and evaporated under reduced pressure to afford a crude oil that was purified by passing it through a silica gel cartridge (eluent gradient: from hexane to hexane/ethyl acetate 8:2). 132 mg of (S)-3-[4-(4-fluorophenyl)-oxazol-2-yl]-piperidine-1-carboxylic acid benzyl ester were obtained as a pale yellow oil that solidified on standing.
Yield: 35%; LCMS (RT): 6.7 min (Method F): MS (ES+) gave m/z: 381.0.

4(B) (S)-3-[4-(4-Fluoro-phenyl)-oxazol-2-yl]-piperidine

Pd/C (10%, 20 mg) was added to a solution of (S)-3-[4-(4-fluoro-phenyl)-oxazol-2-yl]-piperidine-1-carboxylic acid benzyl ester (105 mg, 0.276 mmol) and 1N HCl (276 uL) in EtOH (25 ml). The mixture was hydrogenated at 25 psi at room temperature for 2 h, the catalyst was filtered off and the filtrate was evaporated under reduced pressure. The crude residue was dissolved in MeOH and loaded onto a SCX cartridge. After elution with EtOH and then MeOH, the title compound was recovered pure by eluting with 2% $NH_3$ in MeOH.

(S)-3-[4-(4-Fluoro-phenyl)-oxazol-2-yl]-piperidine (55 mg) was obtained as a pale oil. Yield: 81%; LCMS (RT): 2.9 min (Method F): MS (ES+) gave m/z: 247.0.

4(C) (6-Fluoro-pyridin-3-yl)-{(S)-3-[4-(4-fluoro-phenyl)-oxazol-2-yl]-piperidin-1-yl}- methanone

A mixture of 6-fluoro-nicotinic acid (37 mg, 0.26 mmol), EDC (58 mg, 0.3 mmol), HOAT (41 mg, 0.3 mmol) in dichloromethane (10 ml) was stirred for 10 min at room temperature; then a solution of (S)-3-[4-(4-fluoro-phenyl)-oxazol-2-yl]-piperidine (55 mg, 0.22 mmol) in dichloromethane (5 ml) was added. After stirring for 2 h at room temperature, the solvent was evaporated, the residue was partitioned between ethyl acetate and 0.2M NaOH (aq); the organic phase was separated, washed with water, dried over $Na_2SO_4$ and concentrated. The crude was purified by flash chromatography (silica gel cartridge, eluent gradient: from ethyl acetate/hexane 1:9 to ethyl acetate/hexane 6:4) to give 67 mg of pink solid. Yield: 83%; [$\alpha_D$]= +105° (c=0.5, MeOH); LCMS (RT): 2.91 min (Method H); MS (ES+) gave m/z: 370.1 (MH+).
$^1$H-NMR (DMSO-$d_6$, 353K), δ (ppm): 8.38 (s, 1H) 8.27-8.31 (m, 1H) 8.01 (td, 1H) 7.74-7.81 (m, 2H) 7.18-7.27 (m, 3H) 4.18 (br. s., 1H) 3.76 (br. s., 1H) 3.49 (dd, 1H) 3.33 (ddd, 1H) 3.14-3.24 (m, 1H) 2.16-2.26 (m, 1H) 1.77-2.02 (m, 2H) 1.57-1.72 (m, 1H).

Example 5

(2-Fluoro-pyridin-4-yl)-{(S)-3-[4-(4-fluoro-phenyl)-oxazol-2-yl]-piperidin-1-yl} - methanone

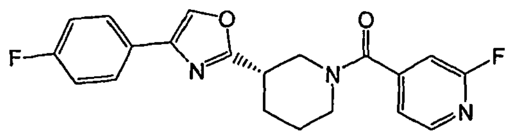

(2-Fluoro-pyridin-4-yl)-{(S)-3-[4-(4-fluoro-phenyl)-oxazol-2-yl]-piperidin-1-yl}-methanone was prepared following the same procedure described in Example 4(C), starting from (S)-3-[4-(4-fluoro-phenyl)-oxazol-2-yl]-piperidine, prepared as described in Example 4(B), and using 2-fluoro-pyridine-4-carboxylic acid as the acid of choice.
Yield: 100% (pale gum); LCMS (RT): 2.93 min (Method H); MS (ES+) gave m/z: 370.1 (MH+).
$^1$H-NMR (DMSO-$d_6$, 353K), δ (ppm): 8.38 (s, 1H) 8.29-8.35 (m, 1H) 7.73-7.84 (m, 2H) 7.32 (ddd, 1H) 7.18-7.28 (m, 2H) 7.12-7.17 (m, 1H) 4.13 (br. s., 1H) 3.69 (br. s., 1H) 3.47 (dd, 1H) 3.26-3.38 (m, 1H) 3.20 (ddd, 1H) 2.14-2.25 (m, 1H) 1.76-2.01 (m, 2H) 1.52-1.73 (m, 1H).

Example 6

(3-Fluoro-pyridin-4-yl)-{(S)-3-[4-(4-fluoro-phenyl)-oxazol-2-yl]-piperidin-1-yl}-methanone

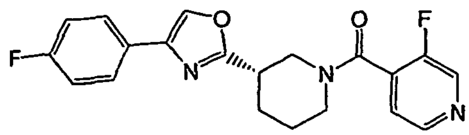

A mixture of 3-fluoro-isonicotinic acid (34 mg, 0.24 mmol), EDC (69 mg, 0.36 mmol), HOBT (37 mg, 0.24 mmol) and triethylamine (84 uL, 0.6 mmol) in dioxane (5 ml) was stirred for 30 min at room temperature; then a solution of (S)-3-[4-(4-fluorophenyl)-oxazol-2-yl]-piperidine (68 mg, 0.275 mmol), prepared as described in Example 4(B), in dioxane (5 ml) was added. After stirring for 6 h at room temperature, the solvent was evaporated, the residue was partitioned between ethyl acetate and citric acid (aq.); the organic phase was separated, washed with 1N NaOH, dried over $Na_2SO_4$ and concentrated under reduced pressure. The crude was purified by flash chromatography (silica gel, eluent gradient: from ethyl acetate/petroleum ether 3:7 to ethyl acetate/petroleum ether 1:1) to give 58 mg of pale yellow gummy solid. Yield: 83%; [$\alpha_D$]= +93.6° (c=1.05, MeOH); LCMS (RT): 2.73 min (Method H); MS (ES+) gave m/z: 370.2 (MH+).
$^1$H-NMR (DMSO-$d_6$, 353K), δ (ppm): 8.64 (s, 1H) 8.51 (dd, 1H) 8.38 (br. s., 1H) 7.78 (br. s., 2H) 7.43 (t, 1H) 7.15-7.29 (m, 2H) 4.51 (br. s., 1 H) 4.04 (br. s., 1H) 3.30-3.55 (m, 2H) 3.11 - 3.28 (m, 1H) 2.15 - 2.28 (m, 1H) 1. 78-2.02 (m, 2H)

1.47-1.70 (m, 1H).

Example 7

(S)-(3-(4-(4-Fluoro-phenyl)-oxazol-2-yl)-piperidin-1-yl)(5-methyl-isoxazol-4-yl)-methanone

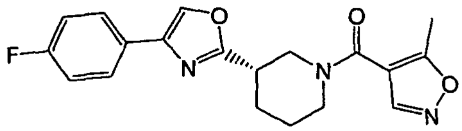

A mixture of 5-methylisoxazole-4-carboxylic acid (32 mg, 0.25 mmol), EDC (48 mg, 0.25 mmol), HOAT (34 mg, 0.25 mmol) in dioxane (5 ml) was stirred for 30 min at room temperature; then a solution of (S)-3-[4-(4-fluoro-phenyl)-oxazol-2-yl]-piperidine (41 mg, 0.167 mmol) in dioxane (5 ml) was added. After stirring overnight at room temperature, the solvent was evaporated, the residue was partitioned between ethyl acetate and 5% citric acid (aq.); the organic phase was separated, dried over $Na_2SO_4$ and concentrated. The crude was purified by flash chromatography (silica gel cartridge, eluent gradient: from petroleum ether to ethyl acetate/petroleum ether 1:1) to give 31 mg of gummy white solid.
Yield: 52%; LCMS (RT): 2.91 min (Method H); MS (ES+) gave m/z: 356.1 (MH+).
$^1$H-NMR (DMSO-$d_6$, 353K), δ (ppm): 8.57 (s, 1H) 8.38 (s, 1H) 7.73-7.81 (m, 2H) 7.18-7.26 (m, 2H) 4.20 (dd, 1H) 3.78 (dt, 1H) 3.49 (dd, 1H) 3.32 (ddd, 1H) 3.10-3.21 (m, 1H) 2.45 (s, 3H) 2.14-2.28 (m, 1H) 1.90-2.02 (m, 1H) 1.77-1.90 (m, 1H) 1.53-1.72 (m, 1H).

Example 8

(S)-(4-Fluoro-phenyl)(3-(4-(pyridin-2-yl)-oxazol-2-yl)-piperidin-1-yl)-methanone

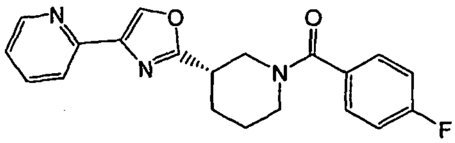

A solution of (S)-1-(4-fluoro-benzoyl)-piperidine-3-carboxylic acid amide (0.2 g, 0.8 mmol), prepared as described in Example 1(C), and 2-(bromoacetyl)-pyridine hydrobromide (90 mg, 0.32 mmol) in dry N-methyl-2-pyrrolidinone (2.5 mL) was heated at 100°C for 5 h. The reaction mixture was cooled to room temperature, ethyl acetate was added and the organic layer was washed sequentially with water (twice) and with brine (twice). The organics were dried over sodium sulphate and evaporated under reduced pressure to afford a crude oil that was purified by flash chromatography: after 3 successive column chromatography purifications (silica gel, eluent: DCM/MeOH/$NH_4OH$ 98:2:0.2), 18 mg of (S)-(4-Fluoro-phenyl)(3-(4-(pyridin-2-yl)-oxazol-2-yl)-piperidin-1-yl)-methanone were obtained as a brown oil.
Yield: 16%; LCMS (RT): 1.99 min (Method H); MS (ES+) gave m/z: 352.2 (MH+).
$^1$H-NMR (DMSO-$d_6$, 353K), δ (ppm): 8.57 (ddd, 1H) 8.43 (s, 1H) 7.77-7.88 (m, 2H) 7.43-7.50 (m, 2H) 7.28-7.33 (m, 1H) 7.19-7.27 (m, 2H) 4.21 (dd, 1H) 3.78 (dd, 1H) 3.46 (dd, 1H) 3.13-3.35 (m, 2H) 2.15-2.28 (m, 1H) 1.78-2.01 (m, 2H) 1.52-1.70 (m, 1H).

Example 9

(S)-(3,4-Difluoro-phenyl)(3-(4-(pyridin-2-yl)-oxazol-2-yl)-piperidin-1-yl)-methanone

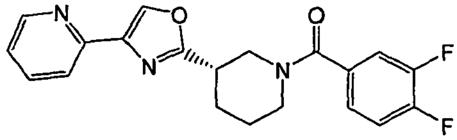

9(A) (S)- 1-(3,4-Difluoro-benzoyl)-piperidine-3-carboxylic acid amide

To a suspension of (S)-piperidine-3-carboxylic acid amide hydrochloride (2.3 g, 14 mmol), prepared as described in Example 1(B), in dry dichloromethane (50 mL), triethylamine (4.9 mL, 35 mmol) and 3,4-difluorobenzoyl chloride (1.93 mL, 15.4 mmol) were added dropwise at 0°C. The reaction mixture was allowed to warm at room temperature and stirred under nitrogen atmosphere for 14h. The solution was washed with 5% citric acid (aq.), with 1N NaOH, then with brine and the organic layer was dried over $Na_2SO_4$ and evaporated under reduced pressure. The crude was purified by trituration from DCM/hexane 1:1 to give 2.5 g of (S)-1-(3,4-difluorobenzoyl)-piperidine-3-carboxylic acid amide.
Yield: 67%; LCMS (RT): 3.1 min (Method F); MS (ES+) gave m/z: 269.0.

9(B) (S)-(3,4-Difluoro-phenyl)(3-(4-(pyridin-2-yl)-oxazol-2-yl)-piperidin-1-yl)-methanone

A solution of (S)-1-(3,4-difluoro-benzoyl)-piperidine-3-carboxylic acid amide (0.214 g, 0.8 mmol) and 2-(bromoacetyl)-pyridine hydrobromide (90 mg, 0.32 mmol) in dry N-methyl-2-pyrrolidinone (3 mL) was heated at 110°C for 7 h. The reaction mixture was cooled to room temperature, ethyl acetate was added and the organic layer was washed sequentially with water (twice) and with brine (twice). The organics were dried over sodium sulphate and evaporated under reduced pressure to afford a crude oil that was purified by flash chromatography (silica gel, eluent gradient: from DCM/MeOH/$NH_4OH$ 99:1:0.1 to DCM/MeOH/$NH_4OH$ 98:2:0.2). The solid that was recovered from this purification was purified again by flash chromatography (silica gel, eluent: DCM/MeOH/$NH_4OH$ 99:1:0.1) to afford 8.5 mg of (S)-(3,4-difluorophenyl)(3-(4-(pyridin-2-yl)-oxazol-2-yl)-piperidin-1-yl)-methanone, obtained as a yellow gummy solid.
Yield: 7%; LCMS (RT): 4.44 min (Method I); MS (ES+) gave m/z: 370.4 (MH+).
$^1$H-NMR ($CDCl_3$, 328K), δ (ppm): 8.59 (ddd, 1H) 8.17 (s, 1H) 7.85 (ddd, 1H) 7.73 (ddd, 1H) 7.29-7.34 (m, 1H) 7.16-7.25 (m, 3H) 4.29-4.39 (m, 1H) 3.93-4.03 (m, 1H) 3.53 (dd, 1H) 3.27 (ddd, 1H) 3.07-3.18 (m, 1H) 1.83-2.06 (m, 2H) 1.68 (br. s., 1H).

Example 10

(S)-(4-Fluoro-phenyl)(3-(4-(5-fluoro-pyridin-2-yl)-oxazol-2-yl)-piperidin-1-yl)-methanone

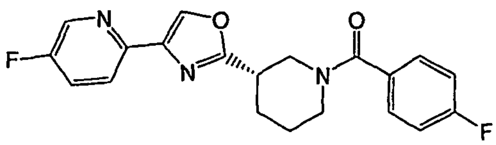

10(A) 5-Fluoro-pyridine-2-carbonitrile

A solution of 2-bromo-5-fluoro-pyridine (5.0 g, 28.4 mmol), CuCN (2.01 g, 22.5 mmol) and NaCN (1.14 g, 23.2 mmol) in dry DMF (50 ml) was refluxed for 9h. The reaction mixture was allowed to cool down to room temperature and a solution of 2% $K_2CO_3$ (aq.) was added. Ethyl acetate was added and the phases were separated. The organic layer was dried over sodium sulphate and evaporated under reduced pressure to give a crude solid that was triturated from hexane.
Yield: 50%; LCMS (RT): 2.5 min (Method G); MS (ES+) gave m/z: 122.9 (MH+).

10(B) 1-(5-Fluoro-pyridin-2-yl)-ethanone

To a solution of 5-fluoro-pyridine-2-carbonitrile (2.6 g, 21.31 mmol) in dry THF (50 ml), cooled at -20°C, under nitrogen atmosphere, methylmagnesium bromide (3M solution in diethyl ether, 7.1 ml, 21.31 mmol) was added dropwise. After stirring overnight at -20°C, the reaction mixture was slowly allowed to warm to room temperature, and then a saturated solution of $NH_4Cl$ (aq.) was added to adjust the pH to 2. Ethyl acetate was added and the phases were

separated. Evaporation of the solvent gave a crude solid that was purified through a silca gel cartridge (eluent: DCM/ petroleum ether 1:1). The solid that was recovered from this purification was purified again by flash chromatography (silica gel, eluent: diethyl ether/petroleum ether 1:9) to afford 1 g of 1-(5-fluoro-pyridin-2-yl)-ethanone.
Yield: 34%; LCMS (RT): 3.4 min (Method F); MS (ES+) gave m/z: 140.0 (MH+).

10(C) 2-Bromo-1-(5-fluoro-pyridin-2-yl)-ethanone hydrobromide

To a solution of 1-(5-fluoro-pyridin-2-yl)-ethanone (200 mg, 1.439 mmol) in 33% HBr in acetic acid (0.7 ml), cooled at 0°C, a suspension of pyridinium tribromide (665 mg, 1.87 mmol) in acetic acid (14 ml) was added. After stirring at room temperature for 3.5 h, 50 ml of diethyl ether were added and the reaction mixture was kept overnight at -4°C in the refrigerator. The pale yellow solid that precipitated out was filtered (218 mg). LC-MS analysis showed that the yellow solid is pure 2-bromo-1-(5-fluoro-pyridin-2-yl)-ethanone hydrobromide. The filtrate was evaporated under reduced pressure and the crude solid was then triturated from petroleum ether to give another 280 mg of pure 2-bromo-1-(5-fluoro-pyridin-2-yl)-ethanone hydrobromide.
Yield: quantitative; LCMS (RT): 4.6 min (Method F); MS (ES+) gave m/z: 218.0 and 220.0 (MH+).

10(D) (S)-(4-Fluoro-phenyl)(3-(4-(5-fluoro-pyridin-2-yl)-oxazol-2-yl)-piperidin-1-yl)-methanone

A solution of (S)-1-(4-fluoro-benzoyl)-piperidine-3-carboxylic acid amide (0.35 g, 1.4 mmol), prepared as described in Example 1(C), and 2-bromo-1-(5-fluoro-pyridin-2-yl)-ethanone hydrobromide (218 mg, 1.0 mmol) in dry N-methyl-2-pyrrolidinone (5 mL) was heated at 150°C for 3 h. The reaction mixture was cooled to room temperature, ethyl acetate was added and the organic layer was washed sequentially with water (twice), with 0.2N NaOH (aq.) and with brine (twice). The organics were dried over sodium sulphate and evaporated under reduced pressure to afford a crude oil that was purified by preparative HPLC. The solid that was recovered from this purification was dissolved in ethyl acetate, treated with 0.5N NaOH, and the phases were separated. The organic layer was dried over sodium sulphate and evaporated under reduced pressure to give 7 mg of (S)-(4-fluorophenyl)(3-(4-(5-fluoro-pyridin-2-yl)-oxazol-2-yl)-piperidin-1-yl)-methanone.
Yield: 2%; LCMS (RT): 2.79 min (Method H); MS (ES+) gave m/z: 370.1 (MH+). $^1$H-NMR ($CDCl_3$), δ (ppm): 8.44 (d, 1H) 8.06-8.15 (m, 1H) 7.80-7.91 (m, 1H) 7.39-7.49 (m, 4H) 7.05-7.14 (m, 2H) 4.01 (br. s., 1H) 3.44 (br. s., 1H) 3.14-3.25 (m, 1H) 3.11 (br. s., 1H) 2.27-2.35 (m, 1H) 1.83-2.06 (m, 2H) 1.68 (br. s., 1H).

Example 11

(S)-(4-Fluoro-phenyl)(3-(4-(2-fluoro-phenyl)-oxazol-2-yl)-piperidin-1-yl)-methanone

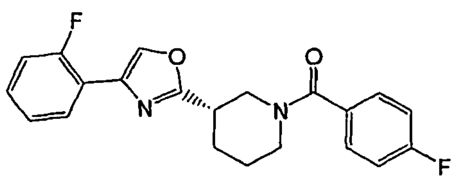

A solution of (S)-1-(4-fluoro-benzoyl)-piperidine-3-carboxylic acid amide (0.161 g, 0.645 mmol), prepared as described in Example 1(C), and 2-fluorophenacyl bromide (100 mg, 0.461 mmol) in dry N-methyl-2-pyrrolidinone (5 mL) was heated at 150°C for 6 h. The reaction mixture was cooled to room temperature, ethyl acetate was added and the organic layer was washed sequentially with water (twice) and with brine (twice). The organics were dried over sodium sulphate and evaporated under reduced pressure to afford a crude oil that was purified by flash chromatography (silica gel, eluent: ethyl acetate/petroleum ether 1:2). 25 mg of (S)-(4-fluorophenyl)(3-(4-(2-fluoro-phenyl)-oxazol-2-yl)-piperidin-1-yl)-methanone were obtained as a colourless gummy solid.
Yield: 15%; LCMS (RT): 3.32 min (Method H); MS (ES+) gave m/z: 369.3 (MH+). $^1$H-NMR (DMSO-$d_6$, 353K), δ (ppm): 8.23 (d, 1H) 7.95 (ddd, 1H) 7.19-7.49 (m, 7H) 4.09-4.33 (m, 1H) 3.77 (ddd, 1H) 3.47 (dd, 1H) 3.14 - 3.32 (m, 2H) 2.17-2.28 (m, 1H) 1.78-2.02 (m, 2H) 1.54-1.71 (m, 1H).

Example 12

(S)-(3-(4-(2-Fluoro-phenyl)-oxazol-2-yl)-piperidin-1-yl)(6-fluoro-pyridin-3-yl)-methanone

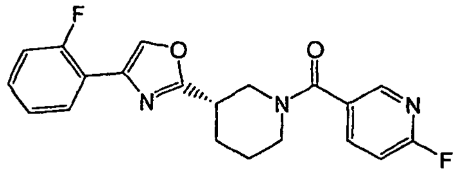

12(A) (S)-3-[4-(2-Fluoro-phenyl)-oxazol-2-yl]-piperidine

The compound was prepared following the procedures described in Examples 4(A) and 4(B), starting from (S)-3-carbamoyl-piperidine-1-carboxylic acid benzyl ester, prepared as described in Example 2(A), and 2-fluorophenacyl bromide.
Yield: 11%; LCMS (RT): 3.2 min (Method F); MS (ES+) gave m/z: 247.2 (MH+).

12(B) (S)-(3-(4-(2-Fluoro-phenyl)-oxazol-2-yl)-piperidin-1-yl)(6-fluoro-pyridin-3-yl)-methanone

The compound was prepared following the same procedure described in Example 6, starting from (S)-3-[4-(2-fluoro-phenyl)-oxazol-2-yl]-piperidine and using 6-fluoronicotinic acid as the acid of choice. Purification was performed by flash chromatography (silica gel, eluent: ethyl acetate/petroleum ether 1:1).
Yield: 51 %; LCMS (RT): 2.37 min (Method H); MS (ES+) gave m/z: 370.2 (MH+). $^1$H-NMR (DMSO-$d_6$, 353K), δ (ppm): 8.30 (ddd, 1H) 8.24 (d, 1H) 8.01 (ddd, 1H) 7.91-7.98 (m, 1H) 7.19-7.42 (m, 4H) 4.10-4.31 (m, 1H) 3.67-3.84 (m, 1H) 3.51 (dd, 1H) 3.18-3.40 (m, 2H) 2.18-2.28 (m, 1H) 1.78-2.03 (m, 2H) 1.58-1.73 (m, 1H).

Example 13

(S)-(3-(4-(2-Fluoro-phenyl)-oxazol-2-yl)-piperidin-1-yl)(2-fluoro-pyridin-4-yl)-methanone

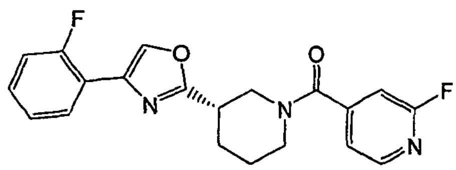

(S)-(3-(4-(2-Fluoro-phenyl)-oxazol-2-yl)-piperidin-1-yl)(2-fluoro-pyridin-4-yl)-methanone was prepared following the same procedure described in Example 6, starting from (S)-3-[4-(2-fluoro-phenyl)-oxazol-2-yl]-piperidine, prepared as described in Example 12(A), and using 2-fluoroisonicotinic acid as the acid of choice. Purification was performed by flash chromatography (silica gel, eluent: ethyl acetate/petroleum ether 4:6).
Yield: 73%; [$\alpha_D$]= +96.15°(c=0.65, MeOH); LCMS (RT): 3.03 min (Method H); MS (ES+) gave m/z: 370.3 (MH+). $^1$H-NMR (DMSO-$d_6$, 353K), δ (ppm): 8.32 (d, 1H), 8.24 (d, 1H), 7.90-7.98 (m, 1H), 7.24-7.42 (m, 4H), 7.12-7.16 (m, 1H), 4.11 (br. s., 1H), 3.70 (br. s., 1H), 3.51 (dd, 1H), 3.19-3.38 (m, 2H), 2.17-2.27 (m, 1H), 1.78-2.03 (m, 2H), 1.58-1.72 (m, 1H).

Example 14

(S)-(3-(4-(2,4-Difluoro-phenyl)-oxazol-2-yl)-piperidin-1-yl)(4-fluoro-phenyl)-methanone

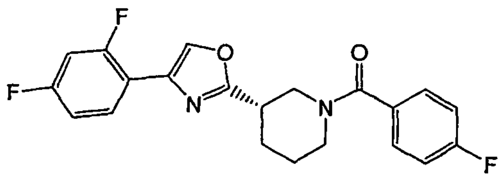

(S)-(3-(4-(2,4-Difluoro-phenyl)-oxazol-2-yl)-piperidin-1-yl)(4-fluoro-phenyl)-methanone was prepared following the same procedure described in Example 11, starting from (S)-1-(4-fluoro-benzoyl)-piperidine-3-carboxylic acid amide, prepared as described in Example 1(C), and 2-bromo-2',4'-difluoro-acetophenone.
Purification was performed by flash chromatography (silica gel, eluent: ethyl acetate/petroleum ether 2:8).
Yield: 24%; [$\alpha_D$]= +93° (c=0.66, MeOH); LCMS (RT): 3.44 min (Method H); MS (ES+) gave m/z: 387.3 (MH+).
$^1$H-NMR (DMSO-$d_6$, 353K), δ (ppm): 8.23 (d, 1H), 7.96 (td, 1H), 7.41-7.49 (m, 2H), 7.13-7.30 (m, 4H), 4.20 (d, 1H), 3.77 (d, 1H), 3.46 (dd, 1H), 3.13-3.32 (m, 2H), 2.16-2.27 (m, 1H), 1.77-2.01 (m, 2H), 1.54-1.70 (m, 1H).

Example 15

(S)-(3-(4-(2,4-Difluoro-phenyl)-oxazol-2-yl)-piperidin-1-yl)(6-fluoro-pyridin-3-yl)-methanone

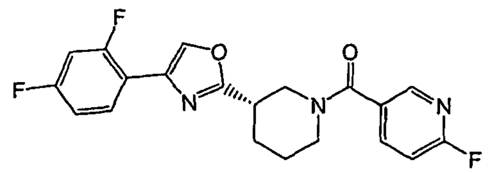

15(A) (S)-3-[4-(2,4-Difluoro-phenyl)-oxazol-2-yl]-piperidine

The compound was prepared following the procedures described in Examples 4(A) and 4(B), starting from (S)-3-carbamoyl-piperidine-1-carboxylic acid benzyl ester, prepared as described in Example 2(A), and 2-bromo-2',4'-difluoroacetophenone.
Yield: 7%; LCMS (RT): 3.4 min (Method F); MS (ES+) gave m/z: 265.1 (MH+).

15(B) (S)-(3-(4-(2,4-Difluoro-phenyl)-oxazol-2-yl)-piperidin-1-yl)(6-fluoro-pyridin-3-yl)-methanone

(S)-(3-(4-(2,4-Difluoro-phenyl)-oxazol-2-yl)-piperidin-1-yl)(6-fluoro-pyridin-3-yl)-methanone was prepared following the same procedure described in Example 6, starting from (S)-3-[4-(2,4-difluoro-phenyl)-oxazol-2-yl)-piperidine and using 6-fluoronicotinic acid as the acid of choice. Purification was performed by flash chromatography (silica gel, eluent: ethyl acetate/petroleum ether 1:1). The solid that was recovered from this purification was purified again by preparative HPLC to give the pure title compound.
Yield: 48%; LCMS (RT): 2.45 min (Method H); MS (ES+) gave m/z: 388.1 (MH+). $^1$H-NMR (DMSO-$d_6$, 353K), δ (ppm): 8.30 (d, 1H), 8.23 (d, 1H), 7.91-8.04 (m, 2H), 7.12-7.31 (m, 3H), 4.20 (br. s., 1H), 3.76 (br. s., 1H), 3.51 (dd, 1H), 3.18-3.40 (m, 2H), 2.14-2.28 (m, 1H), 1.77-2.03 (m, 2H), 1.54-1.74 (m, 1H).

Example 16

(S)-(3-(4-(2,4-Difluoro-phenyl)-oxazol-2-yl)-piperidin-1-yl)(2-fluoro-pyridin-4-yl)-methanone

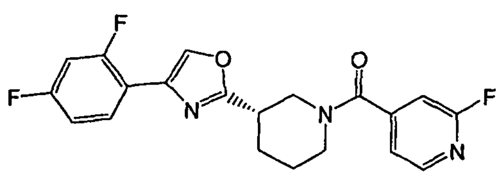

(S)-(3-(4-(2,4-Difluoro-phenyl)-oxazol-2-yl)-piperidin-1-yl)(2-fluoro-pyridin-4-yl)-methanone was prepared following the same procedure described in Example 6, starting from (S)-3-[4-(2,4-difluoro-phenyl)-oxazol-2-yl]-piperidine, prepared as described in Example 15(A), and using 2-fluoroisonicotinic acid as the acid of choice.
Purification was performed by flash chromatography (silica gel, eluent: ethyl acetate/petroleum ether 1:1).
Yield: 92%; $[\alpha_D]$= +82.5°(c=0.7, MeOH); LCMS (RT): 3.08 min (Method H); MS (ES+) gave m/z: 388.2 (MH+).
$^1$H-NMR (DMSO-$d_6$, 353K), δ (ppm): 8.30-8.34 (m, 1H), 8.24 (d, 1H), 7.90-8.04 (m, 1H), 7.23-7.34 (m, 2H), 7.12-7.20 (m, 2H), 4.12 (br. s., 1H), 3.68 (br. s., 1H), 3.49 (dd, 1H), 3.19-3.40 (m, 2H), 2.16-2.28 (m, 1H), 1.77-2.02 (m, 2H), 1.57-1.73 (m, 1 H).

PHARMACOLOGY:

The compounds provided in the present invention are positive allosteric modulators of mGluR5. As such, these compounds do not appear to bind to the orthosteric glutamate recognition site, and do not activate the mGluR5 by themselves. Instead, the response of mGluR5 to a concentration of glutamate or mGluR5 agonist is increased when compounds of Formula I are present. Compounds of Formula I are expected to have their effect at mGluR5 by virtue of their ability to enhance the function of the receptor.

**EXAMPLE A**

**mGluR5 assay on rat cultured cortical astrocytes**

Under exposure to growth factors (basic fibroblast growth factor, epidermal growth factor), rat cultured astrocytes express group I-Gq coupled mGluR transcripts, namely mGluR5, but none of the splice variants of mGluR1, and as a consequence, a functional expression of mGluR5 receptors (Miller et al. (1995) J. Neurosci. 15:6103-9): The stimulation of mGluR5 receptors with selective agonist CHPG and the full blockade of the glutamate-induced phosphoinositide (PI) hydrolysis and subsequent intracellular calcium mobilization with specific antagonist as MPEP confirm the unique expression of mGluR5 receptors in this preparation.
This preparation was established and used in order to assess the properties of the compounds of the present invention to increase the $Ca^{2+}$ mobilization-induced by glutamate without showing any significant activity when applied in the absence of glutamate.

**Primary cortical astrocytes culture:**

Primary glial cultures were prepared from cortices of Sprague-Dawley 16 to 19 days old embryos using a modification of methods described by Mc Carthy and de Vellis (1980) J. Cell Biol. 85:890-902 and Miller et al. (1995) J. Neurosci. 15 (9):6103-9. The cortices were dissected and then dissociated by trituration in a sterile buffer containing 5.36 mM KCl, 0.44 mM $NaHCO_3$, 4.17 mM $KH_2PO_4$, 137 mM NaCl, 0.34 mM $NaH_2PO_4$, 1 g/L glucose. The resulting cell homogenate was plated onto poly-D-lysine precoated T175 flasks (BIOCOAT, Becton Dickinson Biosciences, Erembodegem, Belgium) in Dubelcco's Modified Eagle's Medium (D-MEM GlutaMAX™ I, Invitrogen, Basel, Switzerland) buffered with 25 mM HEPES and 22.7 mM $NaHCO_3$, and supplemented with 4.5g/L glucose, 1 mM pyruvate and 15 % fetal bovine serum (FBS, Invitrogen, Basel, Switzerland), penicillin and streptomycin and incubated at 37°C with 5% $CO_2$. For subsequent seeding, the FBS supplementation was reduced to 10 %. After 12 days, cells were subplated by trypsinisation onto poly-D-lysine precoated 384-well plates at a density of 20.000 cells per well in culture buffer.

**$Ca^{2+}$ mobilization assay using rat cortical astrocytes:**

After one day of incubation, cells were washed with assay buffer containing: 142 mM NaCl, 6 mM KCl, 1 mM $Mg_2SO_4$, 1 mM $CaCl_2$, 20 mM HEPES, 1 g/L glucose, 0.125 mM sulfinpyrazone, pH 7.4. After 60 min of loading with 4 μM Fluo-4 (TefLabs, Austin, TX), the cells were washed three times with 50 μl of PBS Buffer and resuspended in 45 μl of assay Buffer. The plates were then transferred to a Fluorometric Imaging Plate Reader (FLIPR, Molecular Devices, Sunnyvale, CA) for the assessment of intracellular calcium flux. After monitoring the baseline fluorescence for 10 s, a solution containing 10μM of representative compound of the present invention diluted in Assay Buffer (15 μl of 4X dilutions) was added to the cell plate in the absence or in the presence of 300 nM of glutamate. Under these experimental conditions, this concentration induces less than 20 % of the maximal response of glutamate and was the concentration used to detect the positive allosteric modulator properties of the compounds from the present invention. The final DMSO concentration in the assay was 0.3 %. In each experiment, fluorescence was then monitored as a function of time for 3 minutes and the data analyzed using Microsoft Excel and GraphPad Prism. Each data point was also measured two times.
The effect of the compounds of the present invention are performed on primary cortical mGluR5-expressing

cell cultures in the absence or in the presence of 300 nM glutamate. Data are expressed as the percentage of maximal response observed with 30 μM glutamate applied to the cells. Each bar graph is the mean and S.E.M of duplicate data points and is representative of three independent experiments

The compounds of this application have $EC_{50}$ values in the range of less than 10 μM. Example # 1 has $EC_{50}$ value of less than 1 μM.

The results in Example A demonstrate that the compounds described in the present invention do not have an effect per se on mGluR5. Instead, when compounds are added together with an mGluR5 agonist such as glutamate, the effect measured is significantly potentiated compared to the effect of the agonist alone at the same concentration. This data indicates that the compounds of the present invention are positive allosteric modulators of mGluR5 receptors in native preparations.

**EXAMPLE B**

**mGluR5 assay on HEK-expressing rat mGluR5**

**Cell culture**

Positive functional expression of HEK-293 cells stably expressing rat mGluR5 receptor was determined by measuring intracellular $Ca^{2+}$ changes using a Fluorometric Imaging Plate Reader (FLIPR, Molecular Devices, Sunnyvale, CA) in response to glutamate or selective known mGluR5 agonists and antagonists. Rat mGluR5 RT-PCR products in HEK-293 cells were sequenced and found 100% identical to rat mGluR5 Genbank reference sequence (NM_017012). HEK-293 cells expressing rmGluR5 were maintained in media containing DMEM, dialyzed Fetal Bovine Serum (10 %), Glutamax™ (2 mM), Penicillin (100 units/ml), Streptomycin (100 μg/ml), Geneticin (100 μg/ml) and Hygromycin-B (40 μg/ml) at 37°C/5%CO2.

**Fluorescent cell based- $Ca^{2+}$ mobilization assay**

After one day of incubation, cells were washed with assay buffer containing: 142 mM NaCl, 6 mM KCl, 1 mM $Mg_2SO_4$, 1 mM $CaCl_2$, 20 mM HEPES, 1 g/L glucose, 0.125 mM sulfinpyrazone, pH 7.4. After 60 min of loading with 4 uM Fluo-4 (TefLabs, Austin, TX), the cells were washed three times with 50 μl of PBS Buffer and resuspended in 45 μl of assay Buffer. The plates were then transferred to a Fluorometric Imaging Plate Reader (FLIPR, Molecular Devices, Sunnyvale, CA) for the assessment of intracellular calcium flux. After monitoring the baseline fluorescence for 10 seconds, increasing concentrations of representative compound (from 0.01 to 60 μM) of the present invention diluted in Assay Buffer (15 μl of 4X dilutions) was added to the cell. The final DMSO concentration in the assay was 0.3 %. In each experiment, fluorescence was then monitored as a function of time for 3 minutes and the data analyzed using Microsoft Excel and GraphPad Prism. Each data point was also measured two times.

Under these experimental conditions, this HEK-rat mGluR5 cell line is able to directly detect positive allosteric modulators without the need of co-addition of glutamate or mGluR5 agonist. Thus, DFB, CPPHA and CDPPB, published reference positive allosteric modulators that are inactive in rat cortical astrocytes culture in the absence of added glutamate (Liu et al (2006) Eur. J. Pharmacol. 536:262-268; Zhang et al (2005); J. Pharmacol. Exp. Ther. 315:1212-1219) are activating, in this system, rat mGluR5 receptors.

The concentration-response curves of representative compounds of the present invention were generated using the Prism GraphPad software (Graph Pad Inc, San Diego, USA). The curves were fitted to a four-parameter logistic equation:

$$(Y=Bottom + (Top-Bottom)/(1+10^{\wedge}((LogEC50-X)*Hill\ Slope)$$

allowing determination of $EC_{50}$ values.

The Table 1 below represents the mean $EC_{50}$ obtained from at least three independent experiments of selected molecules performed in duplicate.

**Table 1:**

| EXAMPLE # | $Ca^{2+}$ Flux* |
|---|---|
| 1 | ++ |
| 2 | ++ |

(continued)

| EXAMPLE # | $Ca^{2+}$ Flux* |
|---|---|
| 3 | ++ |
| 4 | ++ |
| 5 | ++ |
| 6 | ++ |
| 7 | ++ |
| 8 | ++ |
| 9 | ++ |
| 10 | ++ |
| 11 | ++ |
| 12 | ++ |
| 13 | ++ |
| 14 | ++ |
| 15 | + |
| 16 | ++ |
| ***Table legend:**<br>**(+): 1 μM < $EC_{50}$ <10 μM**<br>**(++): $EC_{50}$ < 1 μM** | |

**EXAMPLE C**

<u>**mGluR5 binding assay**</u>

Activity of compounds of the invention was examined following a radioligand binding technique using whole rat brain and tritiated 2-methyl-6-(phenylethynyl)-pyridine ([$^3$H]-MPEP) as a ligand following similar methods than those described in Gasparini et al. (2002) Bioorg. Med. Chem. Lett. 12:407-409 and in Anderson et al. (2002) J. Pharmacol. Exp. Ther. 303 (3) 1044-1051.

**Membrane preparation:**

Cortices were dissected out from brains of 200-300g Sprague-Dawley rats (Charles River Laboratories, L'Arbresle, France). Tissues were homogenized in 10 volumes (vol/wt) of ice-cold 50 mM Hepes-NaOH (pH 7.4) using a Polytron disrupter (Kinematica AG, Luzern, Switzerland) and centrifuged for 30 min at 40,000 g. (4°C).

The supernatant was discarded and the pellet washed twice by resuspension in 10 volumes 50 mM HEPES-NaOH. Membranes were then collected by centrifugation and washed before final resuspension in 10 volumes of 20 mM HEPES-NaOH, pH 7.4. Protein concentration was determined by the Bradford method (Bio-Rad protein assay, Reinach, Switzerland) with bovine serum albumin as standard.

[$^3$H]-MPEP binding experiments:

Membranes were thawed and resuspended in binding buffer containing 20 mM HEPES-NaOH, 3 mM $MgCl_2$, 3 mM $CaCl_2$, 100 mM NaCl, pH 7.4. Competition studies were carried out by incubating for 1h at 4°C: 3 nM [$^3$H]-MPEP (39 Ci/mmol, Tocris, Cookson Ltd, Bristol, U.K.), 50 μg membrane and a concentration range of 0.003 nM- 30 μM of compounds, for a total reaction volume of 300 μl. The nonspecific binding was defined using 30 μM MPEP. Reaction was terminated by rapid filtration over glass-fiber filter plates (Unifilter 96-well GF/B filter plates, Perkin-Elmer, Schwerzenbach, Switzerland) using 4 x 400 μl ice cold buffer using cell harvester (Filtermate, Perkin-Elmer, Downers Grove, USA). Radioactivity was determined by liquid scintillation spectrometry using a 96-well plate reader (TopCount, Perkin-Elmer, Downers Grove, USA).

**Data analysis:**

The inhibition curves were generated using the Prism GraphPad program (Graph Pad Software Inc, San Diego, USA). $IC_{50}$ determinations were made from data obtained from 8 point-concentration response curves using a non linear regression analysis. The mean of $IC_{50}$ obtained from at least three independent experiments of selected molecules performed in duplicate were calculated.

The compounds of this application have $IC_{50}$ values in the range of less than 30 μM. Example # 1 has $IC_{50}$ value of less than 10 μM.

The results shown in Examples A, B and C demonstrate that the compounds described in the present invention are positive allosteric modulators of rat mGluR5 receptors. These compounds are active in native systems and are able to inhibit the binding of the prototype mGluR5 allosteric modulator [$^3$H]-MPEP known to bind remotely from the glutamate binding site into the transmembrane domains of mGluR5 receptors (Malherbe et al (2003) Mol. Pharmacol. 64(4):823-32).

Thus, the positive allosteric modulators provided in the present invention are expected to increase the effectiveness of glutamate or mGluR5 agonists at mGluR5 receptor. Therefore, these positive allosteric modulators are expected to be useful for treatment of various neurological and psychiatric disorders associated with glutamate dysfunction described to be treated herein and others that can be treated by such positive allosteric modulators.

The compounds of the present invention are allosteric modulators of mGluR5 receptors, they are useful for the production of medications, especially for the prevention or treatment of central nervous system disorders as well as other disorders modulated by this receptor.

The compounds of the invention can be administered either alone, or in combination with other pharmaceutical agents effective in the treatment of conditions mentioned above.

FORMULATION EXAMPLES

Typical examples of recipes for the formulation of the invention are as follows:

1) Tablets

| | |
|---|---|
| Compound of the example 1 | 5 to 50 mg |
| Di-calcium phosphate | 20 mg |
| Lactose | 30 mg |
| Talcum | 10 mg |
| Magnesium stearate | 5 mg |
| Potato starch | ad 200 mg |

In this example, the compound of the example 1 can be replaced by the same amount of any of the described examples 1 to 16.

2) Suspension

An aqueous suspension is prepared for oral administration so that each 1 milliliter contains 1 to 5 mg of one of the described example, 50 mg of sodium carboxymethyl cellulose, 1 mg of sodium benzoate, 500 mg of sorbitol and water ad 1 ml.

3) Injectable

A parenteral composition is prepared by stirring 1.5 % by weight of active ingredient of the invention in 10% by volume propylene glycol and water.

4) Ointment

| | |
|---|---|
| Compound of the example 1 | 5 to 1000 mg |
| Stearyl alcohol | 3 g |
| Lanoline | 5 g |

| | |
|---|---|
| White petroleum | 15 g |
| Water | ad 100 g |

In this example, the compound 1 can be replaced by the same amount of any of the described examples 1 to 16.

Reasonable variations are not to be regarded as a departure from the scope of the invention. It will be obvious that the thus described invention may be varied in many ways by those skilled in the art.

## Claims

**1.** A compound having the general formula I-A

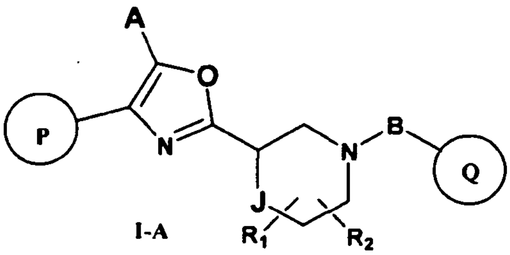

I-A

Wherein

$R_1$ and $R_2$ represent independently hydrogen, -($C_1$-$C_6$)alkyl, -($C_2$-$C_6$)alkenyl, - ($C_2$-$C_6$)alkynyl, arylalkyl, heteroarylalkyl, hydroxy, amino, aminoalkyl, hydroxyalkyl, -($C_1$-$C_6$)alkoxy or $R_1$ and $R_2$ together can form a ($C_3$-$C_7$) cycloalkyl ring, a carbonyl bond C=O or a carbon double bond;

P and Q are each independently selected and denote a cycloalkyl, a heterocycloalkyl, an aryl or heteroaryl group of formula

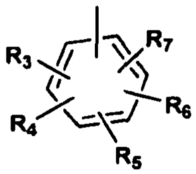 , 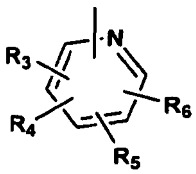 , 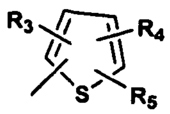 , 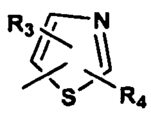 , 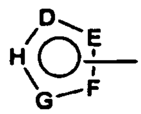

$R_3$, $R_4$, $R_5$, $R_6$, and $R_7$ independently are hydrogen, halogen, -$NO_2$, ($C_1$-$C_6$)alkyl, -($C_3$-$C_6$)cycloalkyl, -($C_3$-$C_7$) cycloalkylalkyl, -($C_2$-$C_6$)alkenyl, -($C_2$-$C_6$)alkynyl, halo-($C_1$-$C_6$)alkyl, heteroaryl, heteroarylalkyl, arylalkyl, aryl, -$OR_8$, -$NR_8R_9$, -C(=$NR_{10}$)$NR_8R_9$, - $NR_8COR_9$, $NR_8CO_2R_9$, $NR_8SO_2R_9$, -$NR_{10}$CO $NR_8R_9$, -$SR_8$, -S(=O)$R_8$, -S (=O)$_2R_8$, -S(=O)$_2NR_8R_9$, -C(=O)$R_8$, -C(=O)-O-$R_8$, -C(=O)$NR_8R_9$, - C(=$NR_8$)$R_9$, or C(=$NOR_8$)$R_9$ substituents; wherein optionally two substituents are combined to the intervening atoms to form a bicyclic heterocycloalkyl, aryl or heteroaryl ring; wherein each ring is optionally further substituted with 1-5 independent halogen, -CN, -($C_1$-$C_6$)alkyl -O-($C_0$-$C_6$)alkyl, -O-($C_3$-$C_7$)cycloalkylalkyl, -O(aryl), - O(heteroaryl), -O-(-$C_1$-$C_3$)alkylaryl, -O-($C_1$-$C_3$)alkylheteroaryl, -N((-$C_1$-$C_6$)alkyl)(($C_0$-$C_3$)alkylaryl) or -N((C0-$C_6$)alkyl)(($C_0$-$C_3$-)alkylheteroaryl) groups;

$R_8$, $R_9$, $R_{10}$ each independently is hydrogen, ($C_1$-$C_6$)alkyl, ($C_3$-$C_6$)cycloalkyl, ($C_3$-$C_7$)cycloalkylalkyl, ($C_2$-$C_6$) alkenyl, ($C_2$-$C_6$)alkynyl, halo-($C_1$-$C_6$)alkyl, heterocycloalkyl, heteroaryl, heteroarylalkyl, arylalkyl or aryl; any of which is optionally substituted with 1-5 independent halogen, -CN, -($C_1$-$C_6$)alkyl, -O-($C_0$-$C_6$)alkyl, -O-($C_3$-$C_7$) cycloalkylalkyl, -O(aryl), -O(heteroaryl), -N($C_0$-$C_6$-alkyl)$_2$,-N(($C_0$-$C_6$)alkyl)(($C_3$-$C_7$-)cycloalkyl) or -N(($C_0$-$C_6$) alkyl)(aryl) substituents;

D, E, F, G and H represent independently -C($R_3$)=, -C($R_3$)=C($R_4$)-,-C(=O)-,-C(=S)-, -O-, -N=, -N($R_3$)- or -S-;

A is hydrogen, ($C_1$-$C_6$)alkyl, ($C_3$-$C_6$)cycloalkyl, ($C_3$-$C_7$)cycloalkylalkyl, ($C_2$-$C_6$)alkenyl, ($C_2$-$C_6$)alkynyl, halo-($C_1$-$C_6$)alkyl, heterocycloalkyl, heteroaryl, heteroarylalkyl, arylalkyl or aryl; any of which is optionally substituted with 1-5 independent halogen, -CN, -($C_1$-$C_6$)alkyl, -O-($C_0$-$C_6$)alkyl, -O-($C_3$-$C_7$)cycloalkylalkyl, -O(aryl), -O (heteroaryl), -N($C_0$-$C_6$-alkyl)$_2$,-N(($C_0$-$C_6$)alkyl)(($C_3$-$C_7$-)Cycloalkyl) or -N(($C_0$-$C_6$)alkyl)(aryl) substituents;

B represents a single bond, -C(=O)-($C_0$-$C_2$)alkyl-, -C(=O)-($C_2$-$C_6$)alkenyl-, -C(=O)-($C_2$-$C_6$)alkynyl-, -C(=O)-O-,

-C(=O)$NR_8$-($C_0$-$C_2$)alkyl-, -C(=$NR_8$)$NR_9$-S(=O)-($C_0$-$C_2$)alkyl-, -S(=O)$_2$-($C_0$-$C_2$)alkyl-, - S(=O)$_2$$NR_8$-($C_0$-$C_2$) alkyl-, C(=$NR_8$)-($C_0$-$C_2$)alkyl-, -C(=$NOR_8$)-($C_0$-$C_2$)alkyl- or -C(=$NOR_8$)$NR_9$-($C_0$-$C_2$)alkyl-;
$R_8$ and $R_9$, independently are as defined above;
J represents a single bond, -C($R_{11}$)($R_{12}$), -O- -N($R_{11}$)- or -S-;
$R_{11}$, $R_{12}$ independently are hydrogen, -($C_1$-$C_6$)alkyl, -($C_3$-$C_6$)cycloalkyl, -($C_3$-$C_7$)cycloalkylalkyl, -($C_2$-$C_6$)alkenyl, -($C_2$-$C_6$)alkynyl, halo($C_1$-$C_6$)alkyl, heteroaryl, heteroarylalkyl, arylalkyl or aryl; any of which is optionally substituted with 1-5 independent halogen, -CN, -($C_1$-$C_6$)alkyl, -O($C_0$-$C_6$)alkyl, -O($C_3$-$C_7$)cycloalkylalkyl, -O(aryl), - O (heteroaryl), -N(($C_0$-$C_6$)alkyl)(($C_0$-$C_6$)alkyl),-N(($C_0$-$C_6$)alkyl)(($C_3$-$C_7$)cycloalkyl) or -N(($C_0$-$C_6$)alkyl)(aryl) substituents;
Any N may be an N-oxide;

or pharmaceutically acceptable salts, hydrates or solvates of such compounds.

2. A compound according to claim 1 having the formula I-B

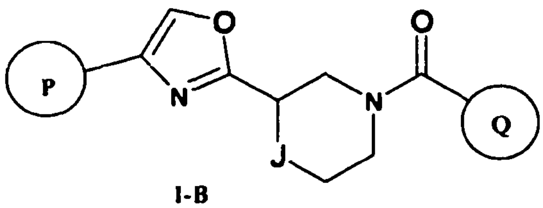

I-B

.

3. A compound according to claim 1, wherein said compound is selected from:

(4-Fluoro-phenyl)-{(S)-3-[4-(4-fluoro-1H-pyrrol-2-yl)-oxazol-2-yl]-piperidin-1-yl}methanone
(6-Fluoro-pyridin-3-yl)-{(S)-3-[4-(4-fluoro-1H-pyrrol-2-yl)-oxazol-2-yl]-piperidin-1-yl}-methanone
(4-Fluoro-phenyl)-{(S)-3-[4-(4-fluorophenyl)-oxazol-2-yl]-piperidin-1-yl}-methanone
(6-Fluoro-pyridin-3-yl)-{(S)-3-[4-(4-fluoro-phenyl)-oxazol-2-yl]-piperidin-1-yl}methanone
(2-Fluoro-pyridin-4-yl)-{(S)-3-[4-(4-fluoro-phenyl)-oxazol-2-yl]-piperidin-1-yl}methanone
(3-Fluoro-pyridin-4-yl)-{(S)-3-[4-(4-fluoro-phenyl)-oxazol-2-yl]-piperidin-1-yl}-methanone
(S)-(3-(4-(4-Fluoro-phenyl)-oxazol-2-yl)-piperidin-1-yl)(5-methyl-isoxazol-4-yl)-methanone
(S)-(4-Fluoro-phenyl)(3-(4-(pyridin-2-yl)-oxazol-2-yl)-piperidin-1-yl)-methanone
(S)-(3,4-Difluoro-phenyl)(3-(4-(pyridin-2-yl)-oxazol-2-yl)-piperidin-1-yl)-methanone
(S)-(4-Fluoro-phenyl)(3-(4-(5-fluoro-pyridin-2-yl)-oxazol-2-yl)-piperidin-1-yl)-methanone
(S)-(4-Fluoro-phenyl)(3-(4-(2-fluoro-phenyl)-oxazol-2-yl)-piperidin-1-yl)-methanone
(S)-(3-(4-(2-Fluoro-phenyl)-oxazol-2-yl)-piperidin-1-yl)(6-fluoro-pyridin-3-yl)-methanone
(S)-(3-(4-(2-Fluoro-phenyl)-oxazol-2-yl)-piperidin-1-yl)(2-fluoro-pyridin-4-yl)-methanone
(S)-(3-(4-(2,4-Difluoro-phenyl)-oxazol-2-yl)-piperidin-1-yl)(4-fluoro-phenyl)-methanone
(S)-(3-(4-(2,4-Difluoro-phenyl)-oxazol-2-yl)-piperidin-1-yl)(6-fluoro-pyridin-3-yl)-methanone and
(S)-(3-(4-(2,4-Difluoro-phenyl)-oxazol-2-yl)-piperidin-1-yl)(2-fluoro-pyridin-4-yl)-methanone.

4. A pharmaceutical composition comprising a therapeutically effective amount of a compound according to claims 1 to 3 and a pharmaceutically acceptable carrier and/or excipient.

5. A compound according to claims 1 to 3 for use in treating or preventing a condition in a mammal, including a human, the treatment or prevention of which is affected or facilitated by the neuromodulatory effect of mGluR5 positive allosteric modulators (enhancer).

6. A compound according to claims 1 to 3 for use in treating or preventing central nervous system disorders selected from the group consisting of anxiety disorders: Agoraphobia, Generalized Anxiety Disorder (GAD), Obsessive-Compulsive Disorder (OCD), Panic Disorder, Posttraumatic Stress Disorder (PTSD), Social Phobia, Other Phobias, Substance-Induced Anxiety Disorder.

7. A compound according to claims I to 3 for use in treating or preventing central nervous system disorders selected from the group consisting of childhood disorders: Attention-Deficit/Hyperactivity Disorder.

**8.** A compound according to claims 1 to 3 for use in treating or preventing central nervous system disorders selected from the group consisting of eating Disorders (Anorexia Nervosa, Bulimia Nervosa).

**9.** A compound according to claims 1 to 3 for use in treating or preventing central nervous system disorders selected from the group consisting of mood disorders: Bipolar Disorders (I & II), Cyclothymic Disorder, Depression, Dysthymic Disorder, Major Depressive Disorder, Substance-Induced Mood Disorder.

**10.** A compound according to claims 1 to 3 for use in treating or preventing central nervous system disorders selected from the group consisting of psychotic disorders: Schizophrenia, Delusional Disorder, Schizoaffective Disorder, Schizophreniform Disorder, Substance-Induced Psychotic Disorder.

**11.** A compound according to claims 1 to 3 for use in treating or preventing central nervous system disorders selected from the group consisting of cognitive disorders: Delirium, Substance-Induced Persisting Delirium, Dementia, Dementia Due to HIV Disease, Dementia Due to Huntington's Disease, Dementia Due to Parkinson's Disease, Dementia of the Alzheimer's Type, Substance-Induced Persisting Dementia, Mild Cognitive Impairment.

**12.** A compound according to claims 1 to 3 for use in treating or preventing central nervous system disorders selected from the group consisting of personality disorders: Obsessive-Compulsive Personality Disorder, Schizoid, Schizotypal disorder.

**13.** A compound according to claims 1 to 3 for use in treating or preventing central nervous system disorders selected from the group consisting of substance-related disorders: Alcohol abuse, Alcohol dependence, Alcohol withdrawal, Alcohol withdrawal delirium, Alcohol-induced psychotic disorder, Amphetamine dependence, Amphetamine withdrawal, Cocaine dependence, Cocaine withdrawal, Nicotine dependence, Nicotine withdrawal, Opioid dependence, Opioid withdrawal.

**14.** A compound according to claims 1 to 3 for use in treating or preventing inflammatory central nervous system disorders selected from multiple sclerosis form such as benign multiple sclerosis, relapsing-remitting multiple sclerosis, secondary progressive multiple sclerosis, primary progressive multiple sclerosis, progressive-relapsing multiple sclerosis.

**Patentansprüche**

**1.** Verbindung der allgemeinen Formel I-A

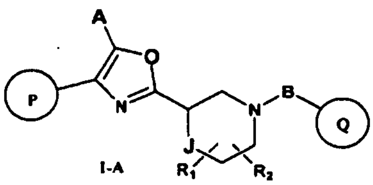

I-A

wobei

$R_1$ und $R_2$ unabhängig Wasserstoff, - $(C_1\text{-}C_6)$ Alkyl, $-(C_2\text{-}C_6)$Alkenyl, $-(C_2\text{-}C_6)$Alkynyl, Arylalkyl, Heteroarylalkyl, Hydroxy, Amino, Aminoalkyl, Hydroxyalkyl, $-(C_1\text{-}C_6)$Alkoxy repräsentieren oder $R_1$ und $R_2$ zusammen einen $(C_3\text{-}C_7)$ Cycloalkylring, eine Carbonylbindung C=O oder eine Kohlenstoffdoppelbindung bilden können;

P und Q jeweils unabhängig ausgewählt sind und eine Cycloalkyl-, eine Heterocycloalkyl-, eine Aryl- oder Heteroarylgruppe der folgenden Formel andeuten

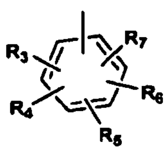 , 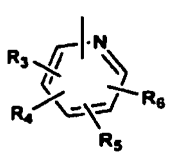 , 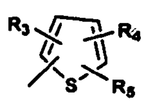 , 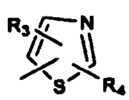 , 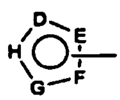

$R_3$, $R_4$, $R_5$, $R_6$ und $R_7$ unabhängig Folgendes sind: Wasserstoff, Halogen, -$NO_2$, -($C_1$-$C_6$)Alkyl, -($C_3$-$C_6$)Cycloalkyl, -($C_3$-$C_7$)Cycloalkylalkyl, -($C_2$-$C_6$)Alkenyl, -($C_2$-$C_6$)Alkynyl, Halo-($C_1$-$C_6$)alkyl, -Heteroaryl, Heteroarylalkyl, Arylalkyl, Aryl, -$OR_8$, -$NR_8R_9$, -C(=$NR_{10}$)$NR_8R_9$, -$NR_8COR_9$, $NR_8CO_2R_9$, $NR_8SO_2R_9$, - $NR_{10}$CO $NR_8R_9$, -$SR_8$, -S(=O)$R_8$, -S (=O)$_2R_8$, -S(=O)$_2NR_8R_9$, - C(=O)$R_8$, -C(=O)-O-$R_8$, -C(=O)$NR_8R_9$, -C(=$NR_8$)$R_9$ oder C(=$NOR_8$)$R_9$ Substituenten; wobei optional zwei Substituenten mit den dazwischen liegenden Atomen kombiniert sind, um einen bicyclischen Heterocycloalkyl-, Aryl oder Heteroarylring zu bilden; wobei jeder Ring optional ferner substituiert ist durch 1-5 unabhängige Halogen, -CN, -($C_1$-$C_6$)Alkyl, -O-($C_0$-$C_6$)Alkyl,- O-($C_3$-$C_7$)Cycloalkylalkyl, -O(Aryl), - O(Heteroaryl), -O-(-$C_1$-$C_3$)Alkylaryl, -O-($C_1$-$C_3$)Alkylheteroaryl, -N((-$C_0$-$C_6$)Alkyl)(($C_0$-$C_3$)alkylaryl) oder -N (($C_0$-$C_6$)Alkyl) (($C_0$-$C_3$-) alkylheteroaryl) Gruppen;

$R_8$, $R_9$, $R_{10}$ jeweils unabhängig Folgendes sind: Wasserstoff, ($C_1$-$C_6$)Alkyl, ($C_3$-$C_6$)Cycloalkyl, ($C_3$-$C_7$)Cycloalkylalkyl, ($C_2$-$C_6$)Alkenyl, ($C_2$-$C_6$)Alkynyl, Halo-($C_1$-$C_6$)alkyl, Heterocycloalkyl, Heteroaryl, Heteroarylalkyl, Arylalkyl oder Aryl; die jeweils optional substituiert sind durch 1-5 unabhängige Halogen, -CN, -($C_1$-$C_6$)Alkyl, -O-($C_0C_6$)Alkyl), - O-($C_3$-$C_7$)Cycloalkylalkyl, -O(Aryl), -O(Heteroaryl), -N($C_0C_6$-Alkyl)$_2$, -N(($C_0$-$C_6$)Alkyl) (($C_3$-$C_7$-)cycloalkyl) oder -N(($C_0$-$C_6$)Alkyl)(aryl) Substituenten;

D, E, F, G und H unabhängig -C($R_3$)=, -C($R_3$)=C($R_4$)-, -C(=O)-, -C(=S)-, -O-, -N=, -N($R_3$)- oder -S- repräsentieren;

A Folgendes ist: Wasserstoff, ($C_1$-$C_6$)Alkyl, ($C_3$-$C_6$)Cycloalkyl, ($C_3$-$C_7$)Cycloalkylalkyl, ($C_2$-$C_6$)Alkenyl, ($C_2$-$C_6$)Alkynyl, Halo-($C_1$-$C_6$)Alkyl, Heterocycloalkyl, Heteroaryl, Heteroarylalkyl, Arylalkyl oder Aryl; die jeweils optional substituiert sind durch 1-5 unabhängige Halogen, -CN, -($C_1$-$C_6$)Alkyl, -O-($C_0$-$C_6$)Alkyl, -O-($C_3$-$C_7$)Cycloalkylalkyl, - O (Aryl), -O(Heteroaryl), -N($C_0$-$C_6$-Alkyl)$_2$, -N(($C_0$-$C_6$)Alkyl)(($C_3$-$C_7$-)cycloalkyl) oder -N(($C_0$-$C_6$)Alkyl)(aryl) Substituenten;

B Folgendes repräsentiert: eine Einfachbindung, -C(=O)-($C_0$-$C_2$)Alkyl-, -C(=O)-($C_2$-$C_6$)Alkenyl-, -C(=O)-($C_2$-$C_6$)Alkynyl-, - C(=O)-O-, -C(=O)$NR_8$-($C_0$-$C_2$)Alkyl-, -C(=$NR_8$)$NR_9$-S(=O)-($C_0$-$C_2$)Alkyl-, -S(=O)$_2$-($C_0$-$C_2$)Alkyl-, -S (=O)$_2NR_8$-($C_0$-$C_2$)Alkyl-, C(=$NR_8$)-($C_0$-$C_2$)Alkyl-, -C(=$NOR_8$)-($C_0$-$C_2$)Alkyl- oder - C (=$NOR_8$)$NR_9$-($C_0$-$C_2$)Alkyl-;

$R_8$ und $R_9$ unabhängig der obigen Definition entsprechen;

J eine Einfachbindung, -C($R_{11}$)($R_{12}$), -O-, -N($R_{11}$)- oder -S-repräsentiert;

$R_{11}$, $R_{12}$ unabhängig Folgendes sind: Wasserstoff, -($C_1$-$C_6$)Alkyl, -($C_3$-$C_6$)Cycloalkyl, -($C_3$-$C_7$)Cycloalkylalkyl, -($C_2$-$C_6$)Alkenyl, -($C_2$-$C_6$)Alkynyl, Halo($C_1$-$C_6$)alkyl, Heteroaryl, Heteroarylalkyl, Arylalkyl oder Aryl; die jeweils optional substituiert sind durch 1-5 unabhängige Halogen, -CN, -($C_1$-$C_6$)Alkyl, -O($C_0$-$C_6$)Alkyl, -O($C_3$-$C_7$)Cycloalkylalkyl, -O(Aryl), -O(Heteroaryl), -N(($C_0$-$C_6$)Alkyl) (($C_0$-$C_6$)alkyl), -N (($C_0$-$C_6$)Alkyl) (($C_3$-$C_7$)cycloalkyl) oder -N (($C_0$-$C_6$) Alkyl) (aryl) Substituenten;

jedes beliebige N ein N-Oxid sein kann;

oder pharmazeutisch akzeptable Salze, Hydrate oder Solvate solcher Verbindungen.

**2.** Verbindung nach Anspruch 1 der Formel I-B

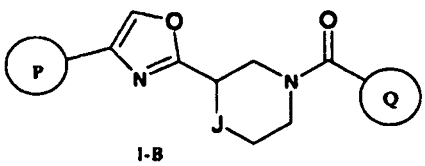

I-B

**3.** Verbindung nach Anspruch 1, wobei die genannte Verbindung ausgewählt ist aus:

(4-Fluor-phenyl)-{(S)-3-[4-(4-fluor-1H-pyrrol-2-yl)-oxazol-2-yl]-piperidin-1-yl}-methanon
(6-Fluor-pyridin-3-yl)-((S)-3-[4-(4-fluor-1H-pyrrol-2-yl)-oxazol-2-yl]-piperidin-1-yl)-methanon
(4-Fluor-phenyl)-{(S)-3-[4-(4-fluorphenyl)-oxazol-2-yl]-piperidin-1-yl}-methanon
(6-Fluor-pyridin-3-yl)-{(S)-3-[4-(4-fluor-phenyl)-oxazol-2-yl]-piperidin-1-yl}-methanon
(2-Fluor-pyridin-4-yl)-{(S)-3-[4-(4-fluor-phenyl)-oxazol-2-yl]-piperidin-1-yl}-methanon
(3-Fluor-pyridin-4-yl)-{(S)-3-[4-(4-fluor-phenyl)-oxazol-2-yl]-piperidin-1-yl}-methanon
(S)-(3-(4-(4-Fluor-phenyl)-oxazol-2-yl)-piperidin-1-yl)(5-methyl-isoxazol-4-yl)-methanon
(S)-(4-Fluor-phenyl)(3-(4-pyridin-2-yl)-oxazol-2-yl)-piperidin-1-yl)-methanon
(S)-(3,4-Difluor-phenyl)(3-(4-(pyridin-2-yl)-oxazol-2-yl)-piperidin-1-yl)-methanon
(S)-(4-Fluor-phenyl)(3-(4-(5-fluor-pyridin-2-yl)-oxazol-2-yl)-piperidin-1-yl)-methanon
(S)-(4-Fluor-phenyl)(3-(4-(2-fluor-phenyl)-oxazol-2-yl)-piperidin-1-yl)-methänon
(S)-(3-(4-(2-Fluor-phenyl)-oxazol-2-yl)-piperidin-1-yl)(6-fluor-pyridin-3-yl)-methanon
(S)-(3-(4-(2-Fluor-phenyl)-oxazol-2-yl)-piperidin-1-yl)(2-fluor-pyridin-4-yl)-methanon
(S)-(3-(4-(2,4-Difluor-phenyl)-oxazol-2-yl)-piperidin-1-yl)(4-fluor-phenyl)-methanon

(S)-(3-(4-(2,4-Difluor-phenyl)-oxazol-2-yl)-piperidin-1-yl)(6-fluor-pyridin-3-yl)-methanon und
(S)-(3-(4-(2,4-Difluor-phenyl)-oxazol-2-yl)-piperidin-1-yl)(2-fluor-pyridin-4-yl)-methanon.

**4.** Pharmazeutische Zusammensetzung, die eine therapeutisch wirksame Menge einer Verbindung nach den Ansprüchen 1 bis 3 und einen pharmazeutisch akzeptablen Träger und/oder Exzipienten umfasst.

**5.** Verbindung nach den Ansprüchen 1 bis 3 zur Verwendung bei der Behandlung oder Verhütung eines Zustands bei einem Säugetier, einschließlich eines Menschen, dessen Behandlung oder Verhütung durch den neuromodulatorischen Effekt von positiven allosterischen mGluR5-Modulatoren (Enhancer) beeinflusst oder erleichtert wird.

**6.** Verbindung nach den Ansprüchen 1 bis 3 zur Verwendung bei der Behandlung oder Verhütung von Störungen des Zentralnervensystems, die ausgewählt sind aus der Gruppe bestehend aus Angststörungen: Agoraphobie, generalisierte Angststörung (GAD), Zwangsstörung (OCD), Panikstörung, posttraumatische Belastungsstörung (PTSD), Sozialphobie, andere Phobien, substanzinduzierte Angststörung.

**7.** Verbindung nach den Ansprüchen 1 bis 3 zur Verwendung bei der Behandlung oder Verhütung von Störungen des Zentralnervensystems, die ausgewählt sind aus der Gruppe bestehend aus Kindheitsstörungen:

Aufmerksamkeitsdefizit/Hyperaktivitätsstörung.

**8.** Verbindung nach den Ansprüchen 1 bis 3 zur Verwendung bei der Behandlung oder Verhütung von Störungen des Zentralnervensystems, die ausgewählt sind aus der Gruppe bestehend aus Essstörungen (Anorexia nervosa, Bulimia nervosa).

**9.** Verbindung nach den Ansprüchen 1 bis 3 zur Verwendung bei der Behandlung oder Verhütung von Störungen des Zentralnervensystems, die ausgewählt sind aus der Gruppe bestehend aus Gemütszustandsstörungen: bipolare Störungen (I & II), zyklothyme Störung, Depressionen, dysthyme Störung, majore depressive Störung, substanzinduzierte Gemütszustandsstörung.

**10.** Verbindung nach den Ansprüchen 1 bis 3 zur Verwendung bei der Behandlung oder Verhütung von Störungen des Zentralnervensystems, die ausgewählt sind aus der Gruppe bestehend aus psychotischen Störungen: Schizophrenie, Wahnstörung, schizoaffektive Störung, schizophreniforme Störung, substanzinduzierte psychotische Störung.

**11.** Verbindung nach den Ansprüchen 1 bis 3 zur Verwendung bei der Behandlung oder Verhütung von Störungen des Zentralnervensystems, die ausgewählt sind aus der Gruppe bestehend aus kognitiven Störungen: Delirium, substanzinduziertes persistierendes Delirium, Demenz, Demenz infolge einer HIV-Erkrankung, Demenz infolge der Huntingtonschen Krankheit, Demenz infolge der Parkinsonschen Krankheit, Demenz vom Alzheimer-Typ, substanzinduzierte persistierende Demenz, leichte kognitive Beeinträchtigung.

**12.** Verbindung nach den Ansprüchen 1 bis 3 zur Verwendung bei der Behandlung oder Verhütung von Störungen des Zentralnervensystems, die ausgewählt sind aus der Gruppe bestehend aus Persönlichkeitsstörungen: zwanghafte Persönlichkeitsstörung, schizoide, schizotypische Störung.

**13.** Verbindung nach den Ansprüchen 1 bis 3 zur Verwendung bei der Behandlung oder Verhütung von Störungen des Zentralnervensystems, die ausgewählt sind aus der Gruppe bestehend aus substanzbezogenen Störungen:

Alkoholmissbrauch, Alkoholabhängigkeit, Alkoholentzug, Alkoholentzugsdelirium, alkoholinduzierte psychotische Störung, Amphetaminabhängigkeit, Amphetaminentzug, Kokainabhängigkeit, Kokainentzug, Nikotinabhängigkeit, Nikotinentzug, Opioidabhängigkeit, Opioidentzug.

**14.** Verbindung nach den Ansprüchen 1 bis 3 zur Verwendung bei der Behandlung oder Verhütung von entzündlichen Zentralnervensystemstörungen, die ausgewählt sind aus Formen der Multiplen Sklerose wie benigne Multiple Sklerose, schubförmig remittierende Multiple Sklerose, sekundär-progressive Multiple Sklerose, primär-progressive Multiple Sklerose, progressiv-schubförmige Multiple Sklerose.

## Revendications

1. Composé ayant la formule générale I-A :

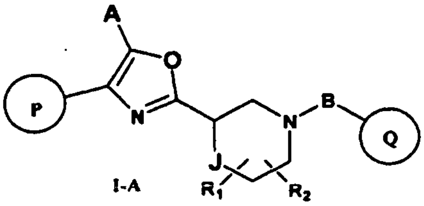

I-A

dans lequel

$R_1$ et $R_2$ représentent indépendamment hydrogène, -($C_1$-$C_6$)alkyle, -($C_2$-$C_6$)alcényle, - ($C_2$-$C_6$)alkynyle, arylalkyle, hétéroarylalkyle, hydroxy, amino, aminoalkyle, hydroxyalkyle, -($C_1$-$C_6$)alkoxy, ou bien $R_1$ et $R_2$ ensemble peuvent former un noyau ($C_3$-$C_7$)cycloalkyle, une liaison carbonyle C=O ou une double liaison carbone ;
P et Q sont sélectionnés indépendamment et dénotent un groupe de formule cycloalkyle, hétérocycloalkyle, aryle ou hétéroaryle

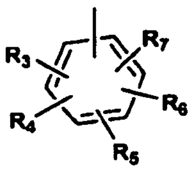 , 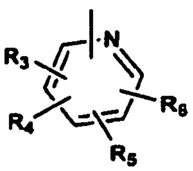 , 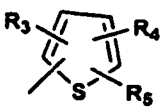 , 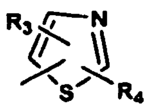 , 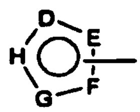

$R_3$, $R_4$, $R_5$, $R_6$, and $R_7$ sont indépendamment des substituants hydrogène, halogène, -$NO_2$, -($C_1$-$C_6$)alkyle, -($C_3$-$C_6$)cycloalkyle, -($C_3$-$C_7$)cycloalkylalkyle, - ($C_2$-$C_6$)alcényle, -($C_2$-$C_6$)alkynyle, halo-($C_1$-$C_6$)alkyle, hétéroaryle, hétéroarylalkyle, arylalkyle, aryle, -$OR_8$, -$NR_8R_9$, -C(=$NR_{10}$)$NR_8R_9$, -$NR_8COR_9$, $NR_8CO_2R_9$, $NR_8SO_2R_9$, -$NR_{10}$CO $NR_8R_9$, -$SR_8$, -S(=O)$R_8$, -S(=O)$_2R_8$, - S(=O)$_2NR_8R_9$, -C(=O)$R_8$, -C(=O)-O-$R_8$, -C(=O)$NR_8R_9$, -C (=$NR_8$)$R_9$, ou C(=$NOR_8$)$R_9$ ; dans lequel deux substituants sont optionnellement combinés aux atomes intermédiaires pour former un noyau bicyclique hétérocycloalkyle, aryle ou hétéroaryle ; dans lequel chaque noyau est optionnellement substitué en outre par 1 à 5 groupes indépendants halogène, -CN, - ($C_1C_6$)alkyle, -O-($C_0$-$C_6$) alkyle, -O-($C_3$-$C_7$)cycloalkylalkyle, -O(aryl), - O(hétéroaryl), -O($C_1$-$C_3$)-alkylaryle, -O-($C_1$-$C_3$)alkylhétéroaryle, -N((-$C_0$-$C_6$)alkyl)(($C_0$-$C_3$)alkylaryl) ou -N(($C_0$-$C_6$-alkyl)($C_0$-$C_3$-alkylhétéroaryl) ;
Chaque $R_8$, $R_9$, $R_{10}$ est indépendamment hydrogène, ($C_1$-$C_6$)alkyle, ($C_3$-$C_6$)cycloalkyle, ($C_3$-$C_7$)cycloalkylalkyle, ($C_2$-$C_6$)alcényle, ($C_2$-$C_6$)alkynyle, halo-($C_1$-$C_6$)alkyle, hétérocycloalkyle, hétéroaryle, hétéroarylalkyle, arylalkyle ou aryle ; l'un quelconque étant optionnellement substitué par 1 à 5 substituants indépendants halogène, -CN, -($C_1$-$C_6$)alkyle, -O-($C_0$-$C_6$)alkyle, -O-($C_3$-$C_7$)cycloalkylalkyle, -O(aryl), -O(hétéroaryl), -N($C_0$-$C_6$-alkyl)$_2$,-N(($C_0$-$C_6$)alkyl)(($C_3$-$C_7$-)cycloalkyl) ou -N($C_0$-$C_6$-alkyl)(aryl) ;
D, E, F, G et H représentent indépendamment -C($R_3$)=, -C($R_3$)=C($R_4$)-,-C(=O)-,-C(=S)-, -O-, -N=, -N($R_3$)- ou -S- ;
A est hydrogène, ($C_1$-$C_6$)alkyle, ($C_3$-$C_6$)cycloalkyle, ($C_3$-$C_7$)cycloalkylalkyle, ($C_2$-$C_6$)alcényle, ($C_2$-$C_6$)alkynyle, halo-($C_1$-$C_6$)alkyle, hétérocycloalkyle, hétéroaryle, hétéroarylalkyle, arylalkyle ou aryle ; l'un quelconque étant optionnellement substitué par 1 à 5 substituants indépendants halogène, -CN, -($C_1$-$C_6$)alkyle, -O-($C_0$-$C_6$)alkyle, -O-($C_3$-$C_7$)cycloalkylalkyle, -O(aryl), - O(hétéroaryl), -N($C_0$-$C_6$-alkyl)$_2$,-N(($C_0$-$C_6$)alkyl)(($C_3$-$C_7$-)cycloalkyl) ou -N(($C_0$-$C_6$)alkyl)(aryl) ;
B représente une simple liaison, -C(=O)-($C_0$-$C_2$)alkyl-, -C(=O)-($C_2$-$C_6$)alcényl-, -C(=O)-($C_2$-$C_6$)alkynyl-, -C (=O)-O-, -C(=O)$NR_8$-($C_0$-$C_2$)alkyl-, -C(=$NR_8$)$NR_9$-S(=O)-($C_0$-$C_2$)alkyl-, -S(=O)$_2$-($C_0$-$C_2$)alkyl-, -S (=O)$_2NR_8$-($C_0$-$C_2$)alkyl-, C(=$NR_8$)-($C_0$-$C_2$)alkyl-, -C(=$NOR_8$)-($C_0$-$C_2$)alkyl- ou -C(=$NOR_8$)$NR_9$-($C_0$-$C_2$)alkyl- ;
$R_8$ et $R_9$, sont indépendamment tels que définis précédemment ;
J représente une simple liaison, -C($R_{11}$)($R_{12}$), -O-, -N($R_{11}$)- ou -S- ;
$R_{11}$, $R_{12}$ sont indépendamment hydrogène, -($C_1$-$C_6$)alkyle, -($C_3$-$C_6$)cycloalkyle, -($C_3$-$C_7$)cycloalkylalkyle, -($C_2$-$C_6$)alcényle, -($C_2$-$C_6$)alkynyle, halo($C_1$-$C_6$)alkyle, hétéroaryle, hétéroarylalkyle, arylalkyle ou aryle ; l'un quelconque étant optionnellement substitué par 1 à 5 substituants indépendants halogène, -CN, -($C_1$-$C_6$)alkyle, -O-($C_0$-$C_6$)alkyle, -0($C_3$-$C_7$)cycloalkylalkyle, -O(aryl),- O(hétéroaryl), -N(($C_0$-$C_6$)alkyl)(($C_0$-$C_6$)alkyl), -N(($C_0$-$C_6$) alkyl)(($C_3$-$C_7$)cycloalkyl) ou -N(($C_0$-$C_6$)alkyl)(aryl) ;

N'importe quel N peut être un N-oxyde ;

ou bien des sels, hydrates ou solvates de qualité pharmaceutique desdits composés.

**2.** Composé selon la revendication 1 ayant la formule I-B

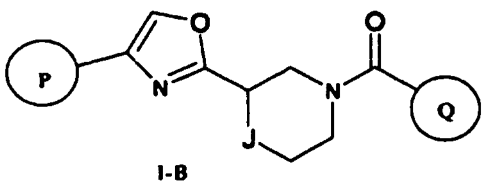

I-B

**3.** Composé selon la revendication 1, dans lequel ledit composé est sélectionné parmi :

(4-Fluoro-phényl)-{(S)-3-[4-(4-fluoro-1H-pyrrol-2-yl)-oxazol-2-yl]-pipéridin-1-yl}-méthanone
(6-Fluoro-pyridin-3-yl)-{(S)-3-[4-(4-fluoro-1H-pyrrol-2-yl)-oxazol-2-yl]-pipéridin-1-yl}-méthanone
(4-Fluoro-phényl)-{(S)-3-[4-(4-fluorophényl)-oxazol-2-yl]-pipéridin-1-yl}-méthanone
(6-Fluoro-pyridin-3-yl)-{(S)-3-[4-(4-fluoro-phényl)-oxazol-2-yl]-pipéridin-1-yl}-méthanone
(2-Fluoro-pyridin-4-yl)-{(S)-3-[4-(4-fluoro-phényl)-oxazol-2-yl]-pipéridin-1-yl}-méthanone
(3-Fluoro-pyridin-4-yl)-{(S)-3-[4-(4-fluoro-phényl)-oxazol-2-yl]-pipéridin-1-yl}-méthanone
(S)-(3-(4-(4-Fluoro-phényl)-oxazol-2-yl)-pipéridin-1-yl)(5-méthyl-isoxazol-4-yl)-méthanone
(S)-(4-Fluoro-phényl)(3-(4-(pyridin-2-yl)-oxazol-2-yl)-pipéridin-1-yl)-méthanone
(S)-(3,4-Difluoro-phényl)(3-(4-(pyridin-2-yl)-oxazol-2-yl)-pipéridin-1-yl)-méthanone
(S)-(4-Fluoro-phényl)(3-(4-(5-fluoro-pyridin-2-yl)-oxazol-2-yl)-pipéridin-1-yl)-méthanone
(S)-(4-Fluoro-phényl)(3-(4-(2-fluoro-phényl)-oxazol-2-yl)-pipéridin-1-yl)-méthanone
(S)-(3-(4-(2-Fluoro-phényl)-oxazol-2-yl)-pipéridin-1-yl)(6-fluoro-pyridin-3-yl)-méthanone
(S)-(3-(4-(2-Fluoro-phényl)-oxazol-2-yl)-pipéridin-1-yl)(2-fluoro-pyridin-4-yl)-méthanone
(S)-(3-(4-(2,4-Difluoro-phényl)-oxazol-2-yl)-pipéridin-1-yl)(4-fluoro-phényl)-méthanone
(S)-(3-(4-(2,4-Difluoro-phényl)-oxazol-2-yl)-pipéridin-1-yl)(6-fluoro-pyridin-3-yl)-méthanone et
(S)-(3-(4-(2,4-Difluoro-phényl)-oxazol-2-yl)-pipéridin-1-yl)(2-fluoro-pyridin-4-yl)-méthanone.

**4.** Composition pharmaceutique comprenant une quantité thérapeutiquement efficace d'un composé selon les revendications 1 à 3 ainsi qu'un vecteur et/ou excipient de qualité pharmaceutique.

**5.** Composé selon les revendications 1 à 3, pour un usage dans le traitement ou la prévention d'une affection chez un mammifère, y compris un être humain, dont le traitement ou la prévention est affecté ou facilité par l'effet neuromodulateur des modulateurs allostériques positifs des mGluR5 (activateur).

**6.** Composé selon les revendications 1 à 3, pour un usage dans le traitement ou la prévention de troubles du système nerveux central sélectionnés parmi le groupe comprenant des troubles d'anxiété : agoraphobie, trouble d'anxiété généralisée (GAD), trouble obsessivo-compulsif (OCD), trouble panique, trouble de stress post-traumatique (PTSD), phobie sociale, autres phobies, trouble d'anxiété induit par substances.

**7.** Composé selon les revendications 1 à 3, pour un usage dans le traitement ou la prévention de troubles du système nerveux central sélectionnés parmi le groupe comprenant des troubles de l'enfance : trouble de déficit de l'attention hyperactivité.

**8.** Composé selon les revendications 1 à 3, pour un usage dans le traitement ou la prévention de troubles du système nerveux central sélectionnés parmi le groupe comprenant des troubles de l'alimentation (anorexie mentale, boulimie mentale).

**9.** Composé selon les revendications 1 à 3, pour un usage dans le traitement ou la prévention de troubles du système nerveux central sélectionnés parmi le groupe comprenant des troubles de l'humeur : troubles bipolaires (I et II), trouble cyclothymique, dépression, trouble dysthymique, trouble dépressif majeur, trouble de l'humeur induit par substances.

**10.** Composé selon les revendications 1 à 3, pour un usage dans le traitement ou la prévention de troubles du système nerveux central sélectionnés parmi le groupe comprenant des troubles psychotiques : schizophrénie, trouble délirant, trouble schizo-affectif, trouble schizophréniforme, trouble psychotique induit par substances.

**11.** Composé selon les revendications 1 à 3, pour un usage dans le traitement ou la prévention de troubles du système nerveux central sélectionnés parmi le groupe comprenant des troubles cognitifs : délire, délire persistant induit par substances, démence, démence due à la maladie du VIH, démence due à la maladie de Huntington, démence due à la maladie de Parkinson, démence de type Alzheimer, démence persistante induite par substances, trouble cognitif léger.

**12.** Composé selon les revendications 1 à 3, pour un usage dans le traitement ou la prévention de troubles du système nerveux central sélectionnés parmi le groupe comprenant des troubles de la personnalité : trouble de la personnalité obsessivo-compulsif, trouble schizoïde, trouble schizotypique.

**13.** Composé selon les revendications 1 à 3, pour un usage dans le traitement ou la prévention de troubles du système nerveux central sélectionnés parmi le groupe comprenant des troubles liés aux substances : abus d'alcool, dépendance à l'alcool, sevrage de l'alcool, délire du sevrage de l'alcool, trouble psychotique induit par l'alcool, dépendance aux amphétamines, sevrage des amphétamines, dépendance à la cocaïne, sevrage de la cocaïne, dépendance à la nicotine, sevrage de la nicotine, dépendance aux opioïdes, sevrage des opioïdes.

**14.** Composé selon les revendications 1 à 3, pour un usage dans le traitement ou la prévention de troubles inflammatoires du système nerveux central sélectionnés parmi de formes de sclérose en plaques telles que sclérose en plaques bénigne, sclérose en plaques rémittente-récurrente, sclérose en plaques secondairement progressive, sclérose en plaques primairement progressive, sclérose en plaques progressive récurrente.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004087048 A
- WO 2005087048 A
- WO 05044797 A1
- WO 06048771 A1
- WO 2007111323 A
- WO 0018744 A


### Non-patent literature cited in the description


- **Nakanishi S et al.** *Brain Res. Rev.,* 1998, vol. 26, 230-235
- **Schoepp DD et al.** *Neuropharmacology,* 1999, vol. 38, 1431-1476
- **Lujan R et al.** *Eur. J. Neurosci.,* 1996, vol. 8, 1488-500
- **Lujan R et al.** *J. Chem. Neuroanat.,* 1997, vol. 13, 219-41
- **Romano C et al.** *J. Comp. Neurol.,* 1995, vol. 355, 455-69
- **Bordi F ; Ugolini A.** *Prog. Neurobiol.,* 1999, vol. 59, 55-79
- **Brauner-Osborne H et al.** *J. Med. Chem.,* 2000, vol. 43, 2609-45
- **Spooren W et al.** *Behav. Pharmacol.,* 2003, vol. 14, 257-77
- **Marino MJ ; Conn PJ.** *Curr. Opin. Pharmacol.,* 2006, vol. 6, 98-102
- **Carlsson A et al.** *Annu. Rev. Pharmacol. Toxicol.,* 2001, vol. 41, 237-260
- **Goff DC ; Coyle JT.** *Am. J. Psychiatry,* 2001, vol. 158, 1367-1377
- **Devon RS et al.** *Mol. Psychiatry.,* 2001, vol. 6, 311-4
- **Ohnuma T et al.** *Brain Res. Mol. Brain Res.,* 1998, vol. 56, 207-17
- **Awad H. et al.** *J. Neurosci.,* 2000, vol. 20, 7871-7879
- **Mannaioni G. et al.** *Neuroscience.,* 2001, vol. 21, 5925-34
- **Pisani A et al.** *Neuroscience,* 2001, vol. 106, 579-87
- **Benquet P. et al.** *J. Neurosci.,* 2002, vol. 22, 9679-86
- **Martin S.J. et al.** *Annu. Rev. Neurosci.,* 2000, vol. 23, 649-711
- **Baudry M. ; Lynch G.** *Neurobiol. Learn. Mem.,* 2001, vol. 76, 284-297
- **Lu et al.** *J. Neurosci.,* 1997, vol. 17, 5196-5205
- **Jia Z. et al.** *Physiol. Behav.,* 2001, vol. 73, 793-802
- **Schulz B et al.** *Neuropharmacology,* 2001, vol. 41, 1-7
- **Rodrigues et al.** *J. Neurosci.,* 2002, vol. 22, 5219-5229
- **Kinney GG et al.** *J. Pharmacol. Exp. Ther.,* 2003, vol. 306 (1), 116-123
- **Lindsley et al.** *Curr. Top. Med. Chem,* 2006, vol. 6, 771-785
- **Knoflach F. et al.** *Proc. Natl. Acad. Sci. USA.,* 2001, vol. 98, 13402-13407
- **Johnson K et al.** *Neuropharmacology,* 2002, vol. 43, 291
- **O'Brien J.A. et al.** *Mol. Pharmacol.,* 2003, vol. 64, 731-40
- **Johnson M.P. et al.** *J. Med. Chem.,* 2003, vol. 46, 3189-92
- **Marino M.J. et al.** *Proc. Natl. Acad. Sci. USA.,* 2003, vol. 100, 13668-73
- **Mitsukawa K. et al.** *Proc Natl Acad Sci U S A,* 2005, vol. 102 (51), 18712-7
- **Wilson J. et al.** *Neuropharmacology,* 2005, vol. 49, 278
- **Mutel V.** *Expert Opin. Ther. Patents,* 2002, vol. 12, 1-8
- **Kew J.N.** *Pharmacol. Ther.,* 2004, vol. 104 (3), 233-44
- **Johnson M.P. et al.** *Biochem. Soc. Trans.,* 2004, vol. 32, 881-7
- **Ritzen A. ; Mathiesen, J.M. ; Thomsen C.** *Basic Clin. Pharmacol. Toxicol.,* 2005, vol. 97, 202-13
- **O'Brien JA et al.** *Mol. Pharmacol.,* 2003, vol. 64, 731-40
- **O'Brien JA.** *J. Pharmacol. Exp. Ther.,* 2004, vol. 309, 568-77
- **Lindsley et al.** *J. Med. Chem.,* 2004, vol. 47, 5825-8
- **Kinney GG et al.** *J. Pharmacol. Exp. Ther.,* 2005, vol. 313, 199-206
- **Balschun D. ; Zuschratter W. ; Wetzel W.** *Neuroscience,* 2006, vol. 142, 691-702

- **ABBADY et al.** *Indian Journal of Chemistry,* 1988, vol. 27B (1), 90-92
- **Green T.W. ; Wuts P.G.M.** Protecting Groups in Organic Synthesis. John Wiley et Sons, 1991
- **Eliel E.L. ; Wilen S.H. ; Mander L.N.** Stereochemistry of Organic Compounds. Wiley-Interscience, 1984
- **Katrizky A.R. ; Rees C.W.** Comprehensive Heterocyclic Chemistry. Pergamon Press, 1984
- **Stahl P.H. ; Wermuth C.G.** Handbook of Pharmaceuticals Salts, Properties, Selection and Use. Wiley, 2002
- **Miller et al.** *J. Neurosci.,* 1995, vol. 15, 6103-9
- **Mc Carthy ; de Vellis.** *J. Cell Biol.,* 1980, vol. 85, 890-902
- **Miller et al.** *J. Neurosci.,* 1995, vol. 15 (9), 6103-9
- **Liu et al.** *Eur. J. Pharmacol.,* 2006, vol. 536, 262-268
- **Zhang et al.** *J. Pharmacol. Exp. Ther.,* 2005, vol. 315, 1212-1219
- **Gasparini et al.** *Bioorg. Med. Chem. Lett.,* 2002, vol. 12, 407-409
- **Anderson et al.** *J. Pharmacol. Exp. Ther.,* 2002, vol. 303 (3), 1044-1051
- **Malherbe et al.** *Mol. Pharmacol.,* 2003, vol. 64 (4), 823-32